# EUROPEAN PATENT SPECIFICATION

(11) **EP 1 309 698 B1**
(45) Date of publication and mention of the grant of the patent: **18.04.2007**
(21) Application number: 01955872.5
(22) Date of filing: 19.07.2001
(51) Int. Cl.: C12N 15/53, C12N 9/02, C12P 7/64

(54) **A CYTOCHROME P450 ENZYME ASSOCIATED WITH THE SYNTHESIS OF DELTA-12 -EPOXY GROUPS IN FATTY ACIDS OF PLANTS**
EIN MIT DER SYNTHESE VON DELTA-12 EPOXY-GRUPPEN IN FETTSÄUREN VON PFLANZEN ASSOZIIERTES CYTOCHROM P450 ENZYM
ENZYME CYTOCHROME P450 ASSOCIEE A LA SYNTHESE DES GROUPES DELTA 12-EPOXYDES DANS LES ACIDES GRAS D'ORIGINE VEGETALE

(30) Priority: 21.07.2000 US 219833 P
(43) Date of publication of application: 14.05.2003
(73) Proprietor: E.I. DU PONT DE NEMOURS AND COMPANY, Wilmington, Delaware 19898 (US)
(72) Inventor: CAHOON, Edgar, B., Wilmington, DE 19806 (US)
(74) Representative: Webber, Philip Michael
(86) International application number: PCT/US2001/022790
(87) International publication number: WO 2002/008269

(56) References cited:
- WO-A-98/46762
- WO-A-98/56922
- WO-A-98/59042
- SEITHER G ET AL: "Isolation of cytochrome P-450 genes from Vernonia galamensis" PHYSIOLOGY, BIOCHEMISTRY AND MOLECULAR BIOLOGY OF PLANT LIPIDS, XX, XX, 1997, pages 389-391, XP002080169
- BLEE ELIZABETH ET AL: "Regio- and stereoselectivity of cytochrome P-450 and peroxygenase-dependent formation of cis-12,13-epoxy-9(Z)-octadecenoic acid (vernolic acid) in Euphorbia lagascae." BIOCHEMICAL AND BIOPHYSICAL RESEARCH COMMUNICATIONS, vol. 197, no. 2, 1993, pages 778-784, XP002211220 ISSN: 0006-291X
- BAFOR M ET AL: "BIOSYNTHESIS OF VERNOLEATE (CIS-12-EPOXYOCTADECA-CIS-9-ENOATE) IN MICROSOMAL PREPARATIONS FROM DEVELOPING ENDOSPERM OF EUPHORBIA LAGASCAE" ARCHIVES OF BIOCHEMISTRY AND BIOPHYSICS, NEW YORK, US, US, vol. 1, no. 303, 15 May 1993 (1993-05-15), pages 145-151, XP001087607 ISSN: 0003-9861
- DATABASE EMBL [Online] 17 July 1998 (1998-07-17) SCHOPFER C.R., ET AL.: "G.max mRNA for putative cytochrome P450, clone CP3" Database accession no. Y10490 XP002211222 -& DATABASE SWISSPROT [Online] 15 December 1998 (1998-12-15) "Cytochrome P450 71D9 (EC 1.14.-.-) (P450 CP3)." Database accession no. O81971 XP002211223
- DATABASE EMBL [Online] 24 January 2000 (2000-01-24) "Capsicum annuum cytochrome P450 (PepCYP) mRNA, complete cds." Database accession no. AF122821 XP002211249
- DATABASE BIOSIS [Online] BIOSCIENCES INFORMATION SERVICE, PHILADELPHIA, PA, US; 1993 VOGEL REINHARD ET AL: "Seed oils for new chemical applications: 1. Vernolic acid produced by Euphorbia lagascae." Database accession no. PREV199396038668 XP002211224 & ANGEWANDTE BOTANIK, vol. 67, no. 1-2, 1993, pages 31-41, ISSN: 0066-1759
- OHLROGGE J.B.: 'Design of newplant products: Engineering of fatty acid metabolism' PLANT PHYSIOLOGY vol. 104, 1994, pages 821 - 826

## Description

### FIELD OF THE INVENTION

This invention is in the field of plant molecular biology. More specifically, this invention pertains to nucleic acid fragments encoding a cytochrome P450 enzyme that catalyzes the synthesis of Δ¹²-epoxy groups in fatty acids of plants and seeds. The products of this enzyme can include epoxy fatty acids such as vernolic acid.

### BACKGROUND OF THE INVENTION

Fatty acids bearing chemical modifications in addition to the common double bonds are found in the storage lipids of many oilseeds (Badami, R.C. and Patil, K.B. (1981) *Prog. Lipid Res. 19*:119-153). Some of these modifications functionalize the fatty acid to produce products that are useful in industrial applications; this is opposed to the more common usage of plant-derived lipids as foods. Examples are the use of the hydroxylated fatty acid ricinoleic acid in lubricants, and the short- or medium-carbon chain length fatty acids from palm oil in detergents. In some cases, fatty acid composition of the storage lipids of oilseeds produced in temperate climates can be modified by the addition of genes from exotic sources so that large amounts of unique fatty acids are produced (Ohlrogge, J. B. (1994) *Plant Physiol. 104,* 821-826).

Epoxidation is among the known modifications to storage lipid fatty acids in plants. Vernolic acid (cis-12-epoxyoctadeca-cis-9-enoic acid) is an example of an epoxy fatty acid found in the seed oils of species such as *Vernonia galamensis* and *Euphorbia lagascae* (Bafor, M. *et al.* (1993) *Arch Biochem Biophys 303*:145-151; and U.S. Patent No. 5,946,784). Vernolic acid can compose as much as 60% of the fatty acids of the seed oils of these plants. Fatty acids such as vernolic acid that contain the epoxy modification may find use as plasticizers, in crosslinking coatings applications, and in setting printing inks (Perdue, R.E. *et al.,* (1986) *Econ. Bot. 40*: 54-68).

Results of previous metabolic studies indicate that the catalytic activity responsible for the introduction of the Δ¹²-epoxy moiety of vernolic acid, found in abundance in *Euphorbia lagascae* seeds, is in the microsomal membrane fraction, most likely the endoplasmic reticulum (Bafor et al. *supra*). This study suggests that the catalytic activity responsible for epoxide formation in this plant is an enzyme of the cytochrome P450 mono-oxygenase class. Genes from *Vernonia galamensis* and *Crepis palaestina* have been isolated, and when expressed in plants or yeast, the encoded proteins are capable of converting linoleic acid to vernolic acid (Lee et al. (1998) *Science* 280:915-918, and U.S. Patent No. 5,846,784). Seither *et al.* (Physiology, Biochemistry and Molecular Biology of Plant lipids, XX, XX, 1997, pp 389-391) relates to the isolation of cytochrome P-450 genes from *Vernonia galamensis.* WO 98/46762 relates to genetic sequences which encode fatty acid Δ 12-epoxygenase enzymes comprising mixed function mono-oxygenase enzymes. In particular, it discloses cDNA sequences which encode plant fatty acid epoxygenases, in particular the *Crepis palaestina* Δ 12-epoxygenase and homologs, analogs and derivatives thereof, and two enzymes from Euphorbia lagascae with putative epoxygenase activity. However, the epoxygenase activities from these other plants producing vernolic acid have been shown to be different from the *Euphorbia lagascae* enzyme. Unlike the *Euphorbia lagascae* enzyme, these enzymes are related to the endoplasmic reticulum-localized fatty acid desaturases (PCT Publication No- WO94/11516, published on May 26, 1994). Apparently these fatty acid epoxidizing enzymes are related in sequence to the class of membrane bound enzymes responsible for fatty acid desaturation and fatty acid hydroxylation (Broun, P. and Somerville, C. (1997) *Plant Physiol 113*: 933-942). Therefore, there are two distinct classes of genes encoding enzymes capable of vernolic acid epoxide group formation, one that is cytochrome P450-dependent, and the other that is related to the fatty acid desaturases and hydroxylases. It appears that the epoxygenase responsible for vernolic acid production in *E. lagascae* developing seeds is a novel and heretofore uncharacterized protein. Isolating and characterizing additional enzymes responsible for Δ¹²-epoxidized fatty acid production will expand the ability to manipulate modified fatty acids in plants. This will further the ongoing work to produce industrially important oils in commercially important crops.

### SUMMARY OF THE INVENTION

The present invention describes an enzyme of the cytochrome P450 mono-oxygenase class that is encoded by a nucleic acid isolated from a *Euphorbia lagascae* developing seed cDNA library. One of the clones, eellc.pk002.i4, when expressed in yeast or unrelated plants, is sufficient to produce novel epoxide containing fatty acids, such as vernolic acid. This is believed to be the first characterization of a plant polynucleotide encoding a cytochrome P450-class enzyme capable of producing Δ¹²-epoxidized fatty acids.

In a first embodiment, an isolated polynucleotide of the claimed invention encodes a polypeptide which is a cytochrome P450 enzyme associated with the synthesis of delta12-epoxy fatty acids wherein said polypeptide has at least 50% identity based on the Clustal method of alignment when compared to a polypeptide of SEQ ID NO:2. In another aspect, this invention also concerns the complement, wherein the complement contains the same number of nucleotides as the isolated polynucleotide sequence and is 100% complementary to the isolated polynucleotide sequence.

In a further embodiment, this invention relates to a chimeric construct comprising the isolated polynucleotide of the present invention that is operably linked to at least one suitable regulatory sequence.

In a further embodiment, the present invention concerns an isolated host cell comprising a chimeric gene of the present invention or an isolated polynucleotide of the present invention. The host cell may be eukaryotic, such as a yeast or a plant cell, or prokaryotic, such as a bacterial cell.

In a further embodiment, the invention also relates to a method of selecting an isolated polynucleotide that affects the level of Δ¹² epoxy fatty acids in a host cell, the method comprising the steps of (a) making an isolated polynucleotide comprising the polynucleotide sequence of the present invention, (b) introducing the isolated polynucleotide into a host cell, and (c) determining presence or absence of Δ¹² epoxy fatty acids in the host cell. It is understood that many organisms can serve as suitable host cells. Useful host cells include, but are not limited to bacteria, yeast, plants, viruses, and animals. A preferred isolated polynucleotide consists of a nucleotide sequence of SEQ ID NO:1.

In further embodiment, this invention relates to a method of obtaining a nucleic acid fragment encoding a cytochrome P450 enzyme associated with the synthesis of Δ¹² epoxy fatty acids comprising the steps of: (a) probing a cDNA or genomic library with an isolated polynucleotide comprising the nucleotide sequence of SEQ ID NO:1 and a complement of such nucleotide sequences; (b) identifying a DNA clone that hybridizes with an isolated polynucleotide of (a) or complement thereof; (c) isolating the identified DNA clone; and (d) sequencing the cDNA or genomic fragment that comprises the isolated DNA clone.

In a further embodiment, the present invention relates to a method for producing Δ¹² epoxy fatty acids in a host cell which comprises, (a) transforming a host cell with the chimeric construct of the present invention, (b) growing the transformed host cells of step (a), and (c) determining the presence or absence of Δ¹² epoxy fatty acids in the transformed cells of (b). As previously mentioned, any host cell can be utilized, with preferred hosts being bacteria, yeast, or plants.

### BRIEF DESCRIPTION OF THE DRAWINGS AND SEQUENCE LISTINGS

The invention can be more fully understood from the following detailed description and the accompanying drawings and Sequence Listing which form a part of this application.

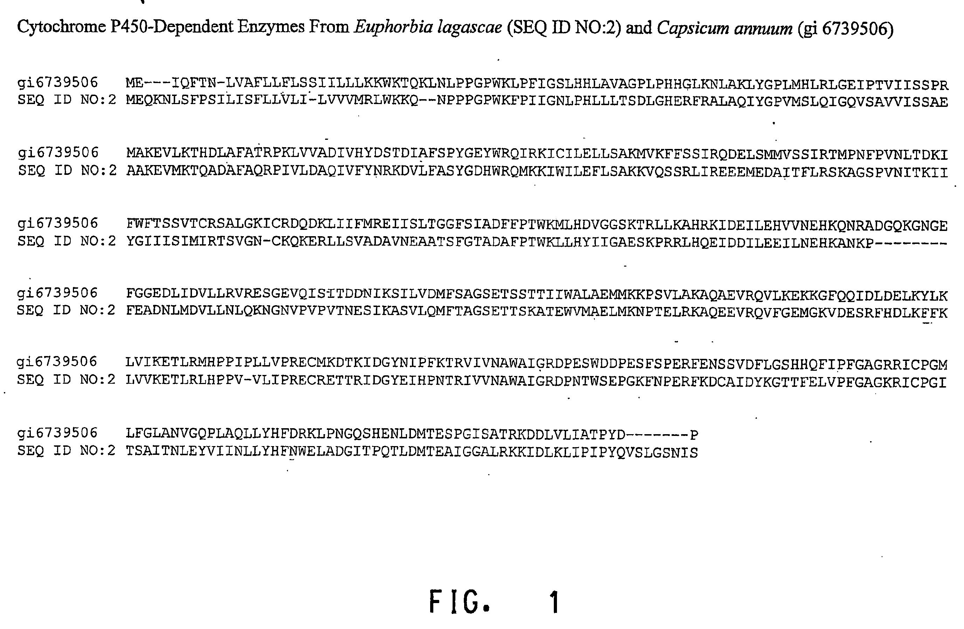

Figure 1 shows a comparison of the amino acid sequences of the *Euphorbia lagascae* enzyme of the present invention (SEQ ID NO:2) and the pepper (*Capsicum annuum*) cytochrome P450 enzyme involved in fungal incompatibility.

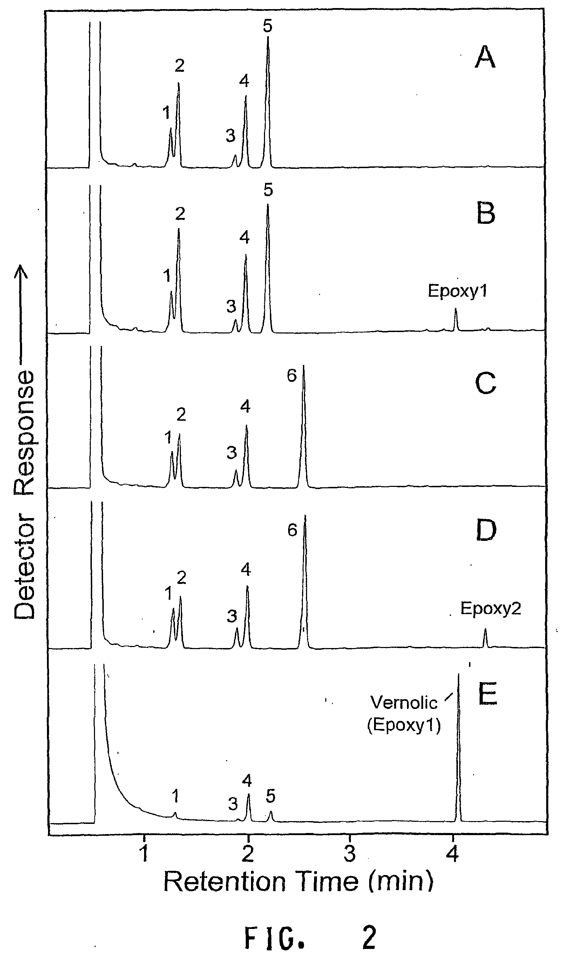

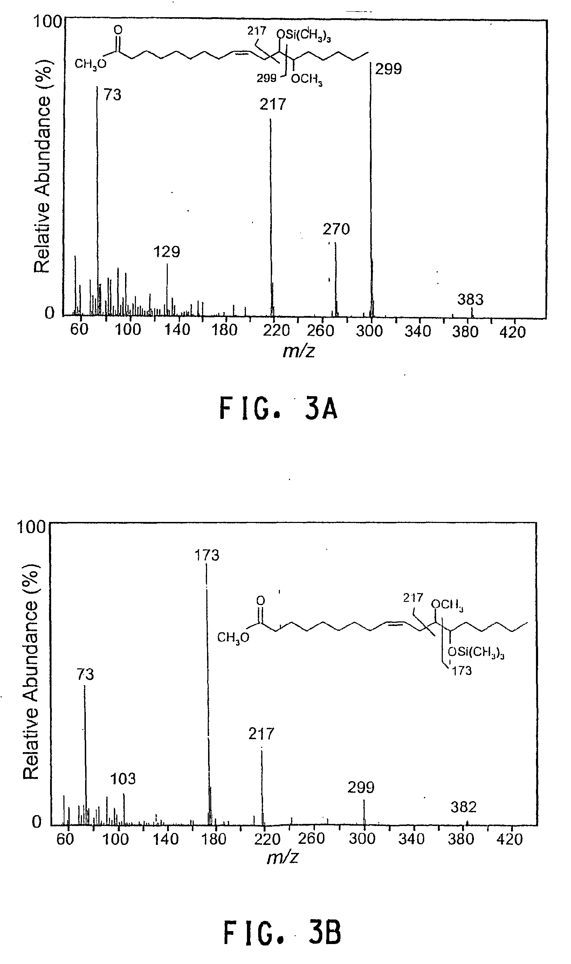

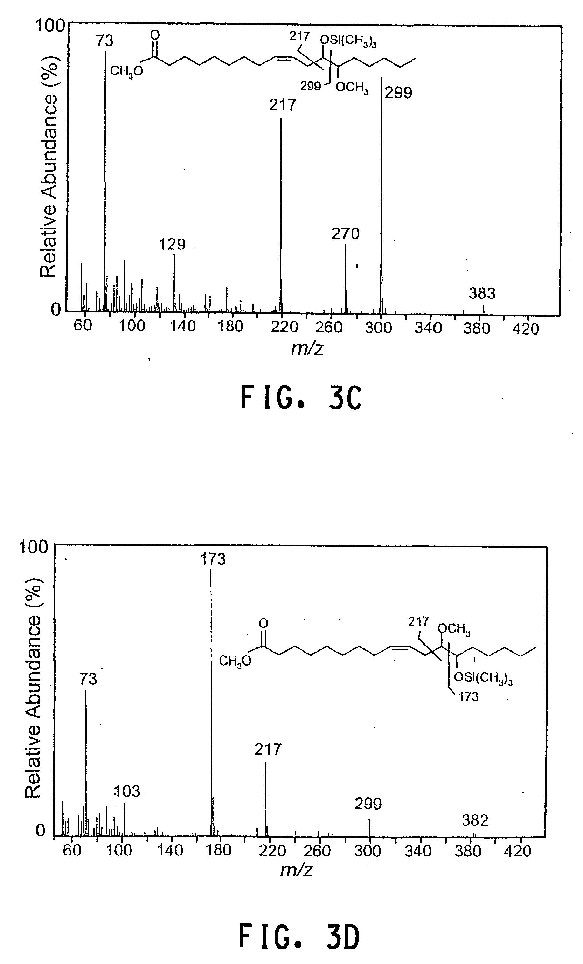

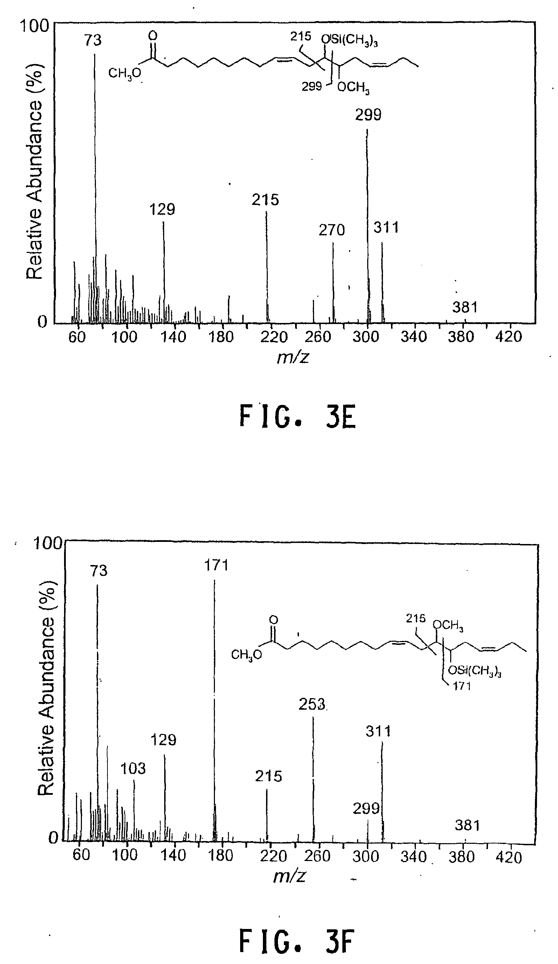

Figure 2. Gas chromatographic analysis of fatty acid methyl esters prepared form *S*. *cerevisiae* cells expressing the *E*. *lagascae* cDNA for EST eellc.pk002.i4 in media supplemented with linoleic or α-linolenic acid. Gas chromatograms in *A* and *C* show fatty acid methyl esters from *S. cerevisiae* cells harboring the expression vector without cDNA insert and grown in the presence of exogenous linoleic and α-linolenic acids, respectively. Gas chromatograms shown in *B* and *D* correspond to fatty acid methyl esters from *S. cerevisiae* cells expressing the *E. lagascae* cDNA for EST eellc.pk002.i4 in media containing linoleic and α-linolenic acids, respectively. The gas chromatogram in *E* corresponds to fatty acid methyl esters prepared from *E. lagascae* seeds. Based on mass spectral analyses shown in Figure 3, the peak labeled "Epoxy1" in *B* is identified as the methyl ester of vernolic acid and the peak labeled "Epoxy2" in *D* is tentatively identified as the methyl ester of Δ¹²-epoxy-octadeca-9,15-dienoic acid. Peaks labeled with numbers correspond to methyl esters of the following fatty acids: *peak 1*, palmitic acid (16:0); *peak 2,* palmitoleic acid (16:1Δ⁹); *peak 3*, stearic acid (18:0); *peak 4,* oleic acid (18:1Δ*⁹*); *peak 5,* linoleic acid (18:2Δ^{9,12}); and *peak 6,* α-linolenic acid (18:3Δ^{9,12,15}).

Figure 3. Mass spectra of derivatives of methyl esters of vernolic acid (*A, B*) and epoxy fatty acids from *S. cerevisiae* cells expressing the *E. lagascae* cDNA for EST eellc.pk002.i4 in media containing linoleic acid (*C, D*) or α-linolenic acid (*E*, *F*)*.* Epoxy fatty acid methyl esters were first reacted in methanolic sulfuric acid, which generates a Δ¹²-hydroxy/Δ¹³-methoxy product and a Δ13-methoxy/Δ¹²-hydroxy product from opening of the epoxy ring. The two products obtained from a given epoxy fatty acid methyl ester were then converted to trimethylsilyl (TMS) derivatives to produce the mass spectra shown in this figure. The mass spectra in *A* and *B* were obtained from derivatives of methyl vernolic acid from *E. lagascae* seed. The mass spectra in *C* and *D* were obtained from derivatives of the fatty acid methyl ester identified as "Epoxy1" in Figure 2. These derivatives were prepared from extracts of *S*. *cerevisiae* cells expressing *E. lagascae* cDNA for EST eellc.pk002.i4 in media containing linoleic acid. The mass spectra in *E* and *F* were obtained from derivatives of the fatty acid methyl ester identified as "Epoxy2" in Figure 2. These derivatives were prepared from extracts of *S*. *cerevisiae* cells expressing *E. lagascae* cDNA in media containing α-linolenic acid. As indicated, these mass spectra are consistent with derivatives of methyl Δ¹²-epoxy-octadeca-9,15-dienoate.

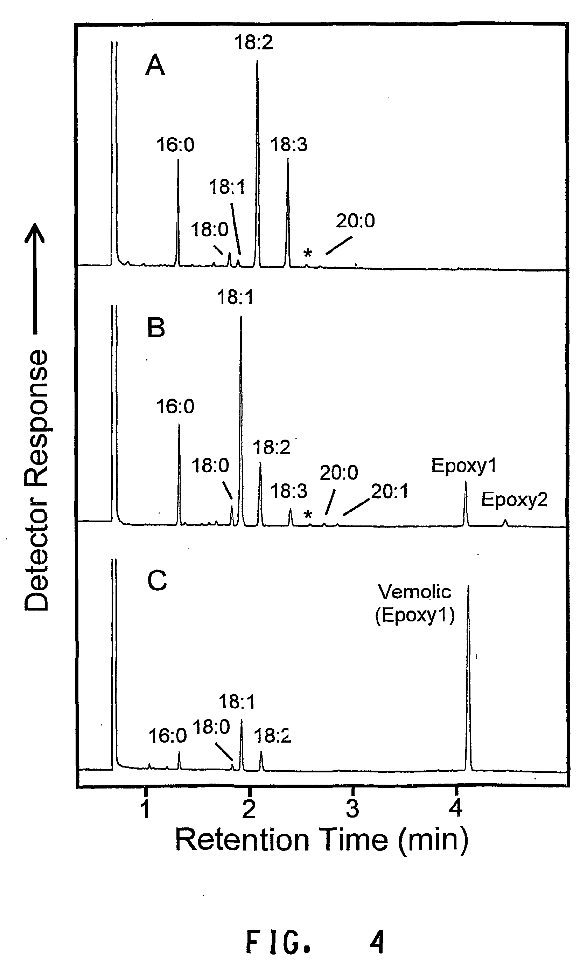

Figure 4. Gas chromatographic analyses of fatty acid methyl esters prepared from tobacco callus transformed with the expression vector (pART/35S) alone (*A*) or with the expression vector containing the open-reading frame of the cDNA for EST eellc.pk002.i4 under control of the cauliflower mosaic virus 35S promoter (*B*). Shown in Panel C are fatty acid methyl esters from *Euphorbia lagascae* seeds. As described in Example 8, the peaks in panel *B* that are labeled "Epoxy1" and "Epoxy2" correspond to the methyl esters of vernolic acid and Δ¹²-epoxy-18:2Δ^{9,15}, respectively. Peaks labeled with asterisks (*) correspond to phytol. Other labeled peaks correspond to methyl esters of the following fatty acids: *16:0*, palmitic acid; *18:0*, stearic acid, *18:1*, oleic acid; *18:2*, linoleic acid; *18:3*, α-linolenic acid; *20:0*, eicosanoic acid; and *20:1*, eicosenoic acid.

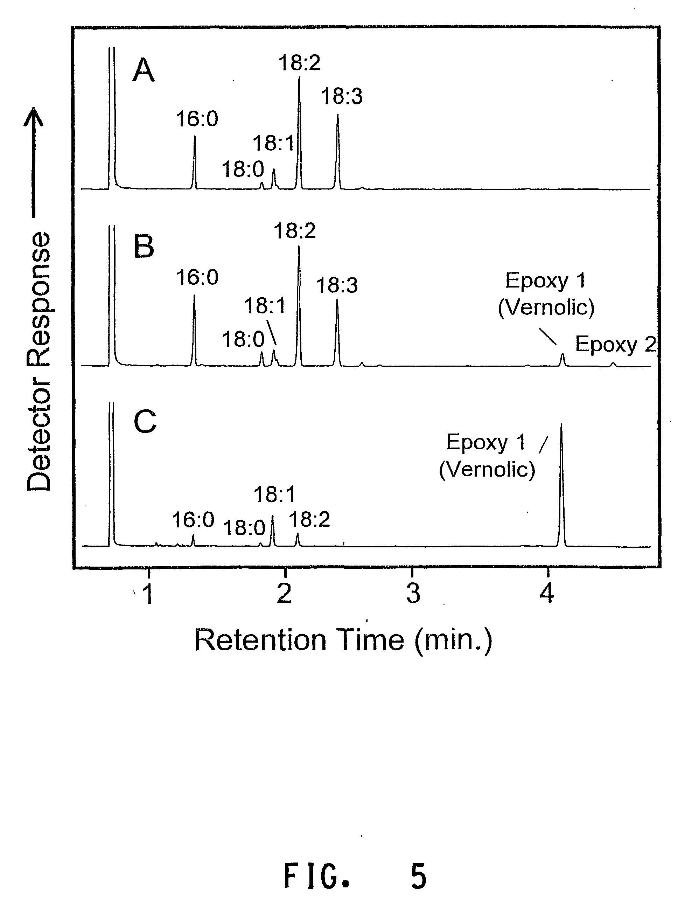

Figure 5. Gas chromatographic analyses of fatty acid methyl esters prepared from an untransformed somatic soybean embryo (*A*) and a transgenic somatic soybean embryo expressing the *Euphorbia lagascae* cytochrome P450 Δ¹²-epoxygenase corresponding to EST eellc.pk002.i4 (*B*). Shown in Panel *C* are fatty acid methyl esters from *Euphorbia lagascae* seeds. As described in Example 9, the peaks in panel *B* that are labeled "Epoxy1" and "Epoxy2" correspond to the methyl esters of vernolic acid and Δ¹²-epox-18:2Δ^{9,15}, respectively. Labeled peaks correspond to methyl esters of the following fatty acids: *16:0,* palmitic acid; *18:0*, stearic acid; *18:1*, oleic acid; *18:2*, linoleic acid; *18:3*, α-linolenic acid.

Table 1 lists the polypeptides that are described herein, the designation of the cDNA clones that comprise the nucleic acid fragments encoding polypeptides representing all or a substantial portion of these polypeptides, and the corresponding identifier (SEQ ID NO:) as used in the attached Sequence Listing.

**TABLE 1**

| A Cytochrome P450 Enzyme Associated With the Synthesis of the Epoxy Group of | | | |
|---|---|---|---|
| Vernolic Acid | | | |
| Plant Species Containing | | SEQ ID NO: | |
| Epoxygenase Activity | Clone Designation | (Nucleotide) | (Amino Acid) |
| *Euphorbia lagascae* | eellc.pk002.i4 | 1 | 2 |

Additional SEQ ID NOs:4-7 represent PCR primers useful for cloning the coding region of SEQ ID NO:1, Details of the strategy can be found in Examples 7 and 8.

The Sequence Listing contains the one letter code for nucleotide sequence characters and the three letter codes for amino acids as defined in conformity with the IUPAC-IUBMB standards described in *Nucleic Acids Res. 13*:3021-3030 (1985) and in the *Biochemical J. 219 (No. 2)*:345-373 (1984).

### DETAILED DESCRIPTION OF THE INVENTION

The terms "epoxy group", "epoxygenated fatty acids", or "the product of an epoxygenase" all refer to the introduction of an epoxide bridge (an oxygen atom covalently bonded to carbon atoms that are in turn covalently bonded to each other, to form a three member ring that is part of a larger molecular structure) at the site of a double bond in the acyl chain of a fatty acid.

"Vernolic acid" refers to the fatty acid 12-epoxyoctadeca-9*cis*-enoic acid which contains eighteen carbon atoms, a *cis* double bond between the 9 and 10 carbon atoms, and an epoxy group between the 12 and 13 carbon atoms. The term "Δ¹²-epoxy fatty acid" refers to a fatty acid such as vernolic acid that contains an epoxy group between the 12 and 13 carbon atoms. The term "cytochrome P450 enzyme associated with the synthesis of Δ¹²-epoxy fatty acids" or "plant epoxygenase" are used interchangeably and are intended to define an enzyme that catalyzes the insertion of an epoxy group at an existing double bond between the 12 and 13 carbon atoms of a fatty acid chain. The substrates for this enzyme can include linoleic and α-linolenic acids, which may be bound to a lipid such as phosphatidylcholine. The products of this enzyme can include Δ¹² epoxy fatty acids such as vernolic acid and 12-epoxyoctadeca-9,15-dienoic acid. Such modified fatty acids are found in the seeds and oils of a limited number of plants including *Euphorbia lagascae, Vernonia,* and *Crepis.* Of these three plants the *Vernonia* and *Crepis* species are known to utilize a divergent desaturase or hydroxylase enzyme to perform the epoxidation (U.S. Patent No. 5,846,784; PCT Application WO 98146762, published on October 22, 1998). In *Euphorbia lagascae,* the conversion of linoleic acid to vernolic acid has been reported to require a cytochrome P450-dependent epoxygenase (Bafor et al. (1993) *Arch Bioch Biophys 303*:145-151).

The terms "polynucleotide", "polynucleotide sequence", "nucleic acid sequence", and "nucleic acid fragment"/"isolated nucleic acid fragment" are used interchangeably herein. These terms encompass nucleotide sequences and the like. A polynucleotide may be a polymer of RNA or DNA, that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. An isolated polynucleotide of the present invention may include at least one of 30 contiguous nucleotides, preferably at least one of 40 contiguous nucleotides, most preferably one of at least 60 contiguous nucleotides derived from SEQ ID N0:1, or the complement of such sequences.

As used herein, an "isolated nucleic acid fragment" is a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

The term "recombinant" means, for example, that a nucleic acid sequence is made by an artificial combination of two otherwise separated segments of sequence, e.g., by chemical synthesis or by the manipulation of isolated nucleic acids by genetic engineering techniques.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of chimeric constructs to produce the desired phenotype in a transformed plant. Chimeric constructs can be designed for use in co-suppression or antisense by linking a nucleic acid fragment or subfragment thereof, whether or not it encodes an active enzyme, in the appropriate orientation relative to a plant promoter sequence.

The terms "homology", "homologous", "substantially similar" and " corresponding substantially" are used interchangeably herein. They refer to nucleic acid fragments wherein changes in one or more nucleotide bases does not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. It is therefore understood, as those skilled in the art will appreciate, that the invention encompasses more than the specific exemplary sequences.

Substantially similar nucleic acid fragments may be selected by screening nucleic acid fragments representing subfragments or modifications of the nucleic acid fragments of the instant invention, wherein one or more nucleotides are substituted, deleted and/or inserted, for their ability to affect the level of the polypeptide encoded by the unmodified nucleic acid fragment in a plant or plant cell. For example, a substantially similar nucleic acid fragment representing at least one of 30 (preferably 40, and more preferably 60) contiguous nucleotides derived from the instant nucleic acid fragment can be constructed and introduced into a plant or plant cell. The level of the polypeptide encoded by the unmodified nucleic acid fragment present in a plant or plant cell exposed to the substantially similar nucleic fragment can then be compared to the level of the polypeptide in a plant or plant cell that is not exposed to the substantially similar nucleic acid fragment.

For example, it is well known in the art that antisense suppression and co-suppression of gene expression may be accomplished using nucleic acid fragments representing less than the entire coding region of a gene, and by using nucleic acid fragments that do not share 100% sequence identity with the gene to be suppressed. Moreover, alterations in a nucleic acid fragment which result in the production of a chemically equivalent amino acid at a given site, but do not effect the functional properties of the encoded polypeptide, are well known in the art. Thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the polypeptide molecule would also not be expected to alter the activity of the polypeptide. Each of the proposed modifications is well within the routine skill in the art, as is determination of retention of biological activity of the encoded products. Consequently, an isolated polynucleotide comprising a nucleotide sequence of at least one of 30 (preferably at least one of 40, most preferably at least one of 60) contiguous nucleotides derived from a nucleotide sequence of SEQ ID NO:1, and the complement of such nucleotide sequences may be used in methods of selecting an isolated polynucleotide that affects the expression of a plant epoxygenase polypeptide in a host cell. A method of selecting an isolated polynucleotide that affects the level of expression of a polypeptide in a virus or in a host cell (eukaryotic, such as plant or yeast, prokaryotic such as bacterial) may comprise the steps of: constructing an isolated polynucleotide of the present invention or an isolated chimeric gene of the present invention; introducing the isolated polynucleotide or the isolated chimeric gene into a host cell; measuring the level of a polypeptide or enzyme activity in the host cell containing the isolated polynucleotide; and comparing the level of a polypeptide or enzyme activity in the host cell containing the isolated polynucleotide with the level of a polypeptide or enzyme activity in a host cell that does not contain the isolated polynucleotide.

Moreover, the skilled artisan recognizes that substantially similar nucleic acid sequences encompassed by this invention are also defined by their ability to hybridize, under moderately stringent conditions (for example, 0.5 X SSC, 0.1 % SDS, 60°C) with the sequences exemplified herein, or to any portion of the nucleotide sequences reported herein and which are functionally equivalent to the promoter of the invention. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions. One set of preferred conditions involves a series of washes starting with 6X SSC, 0.5% SDS at room temperature for 15 min, then repeated with 2X SSC, 0.5% SDS at 45°C for 30 min, and then repeated twice with 0.2X SSC, 0.5% SDS at 50°C for 30 min. A more preferred set of stringent conditions involves the use of higher temperatures in which the washes are identical to those above except for the temperature of the final two 30 min washes in 0.2X SSC, 0.5% SDS was increased to 60°C. Another preferred set of highly stringent conditions involves the use of two final washes in 0.1X SSC, 0.1% SDS at 65°C.

With respect to the degree of substantial similarity between the target (endogenous) mRNA and the RNA region in the construct having homology to the target mRNA, such sequences should be at least 25 nucleotides in length, preferably at least 50, 100, 150, 200, 250, 300, 350, 400, 450, 500, 750, 1000, 1250, or 1500 nucleotides in length; and should be at least 50%, 55%, 60%, 65%, 70%, 75%, 80%, 85%, 90%, or 95% identical.

Substantially similar nucleic acid fragments of the instant invention may also be characterized by the percent identity of the amino acid sequences that they encode to the amino acid sequences disclosed herein, as determined by algorithms commonly employed by those skilled in this art. Suitable nucleic acid fragments (isolated polynucleotides of the present invention) encode polypeptides that are at least 50%, 55%, 60%, 65%, 70%, 75%, 80%, 85%, 90%, or 95% identical to the amino acid sequences reported herein. It is understood that any integer percent identity from 50% to 100% would be suitable for use in the present invention. Suitable nucleic acid fragments not only have the above identities but typically encode a polypeptide having at least 50, 100, 150, 200, 250, 300, 350, 400, 450, or 500 amino acids. Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) *CABIOS.* 5:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5.

A "substantial portion" of an amino acid or nucleotide sequence comprises an amino acid or a nucleotide sequence that is sufficient to afford putative identification of the protein or gene that the amino acid or nucleotide sequence comprises. Amino acid and nucleotide sequences can be evaluated either manually by one skilled in the art, or by using computer-based sequence comparison and identification tools that employ algorithms such as BLAST (Basic Local Alignment Search Tool; Altschul et al. (1993) *J. Mol. Biol. 215*:403-410; see also www.ncbi.nlm.nih.gov/BLAST/). In general, a sequence of ten or more contiguous amino acids or thirty or more contiguous nucleotides is necessary in order to putatively identify a polypeptide or nucleic acid sequence as homologous to a known protein or gene. Moreover, with respect to nucleotide sequences, gene-specific oligonucleotide probes comprising 30 or more contiguous nucleotides may be used in sequence-dependent methods of gene identification (e.g., Southern hybridization) and isolation (e.g., *in situ* hybridization of bacterial colonies or bacteriophage plaques). In addition, short oligonucleotides of 12 or more nucleotides may be used as amplification primers in PCR in order to obtain a particular nucleic acid fragment comprising the primers. Accordingly, a "substantial portion" of a nucleotide sequence comprises a nucleotide sequence that will afford specific identification and/or isolation of a nucleic acid fragment comprising the sequence. The instant specification teaches amino acid and nucleotide sequences encoding polypeptides that comprise one or more particular plant proteins. The skilled artisan, having the benefit of the sequences as reported herein, may now use all or a substantial portion of the disclosed sequences for purposes known to those skilled in this art.

"Codon degeneracy" refers to divergence in the genetic code permitting variation of the nucleotide sequence without effecting the amino acid sequence of an encoded polypeptide. Accordingly, the instant invention relates to any nucleic acid fragment comprising a nucleotide sequence that encodes the amino acid sequences set forth herein. The skilled artisan is well aware of the "codon-bias" exhibited by a specific host cell in usage of nucleotide codons to specify a given amino acid. Therefore, when synthesizing a nucleic acid fragment for improved expression in a host cell, it is desirable to design the nucleic acid fragment such that its frequency of codon usage approaches the frequency of preferred codon usage of the host cell.

"Synthetic nucleic acid fragments" can be assembled from oligonucleotide building blocks that are chemically synthesized using procedures known to those skilled in the art. These building blocks are ligated and annealed to form larger nucleic acid fragments which may then be enzymatically assembled to construct the entire desired nucleic acid fragment. "Chemically synthesized", as related to a nucleic acid fragment, means that the component nucleotides were assembled *in vitro.* Manual chemical synthesis of nucleic acid fragments may be accomplished using well established procedures, or automated chemical synthesis can be performed using one of a number of commercially available machines. Accordingly, the nucleic acid fragments can be tailored for optimal gene expression based on optimization of the nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell where sequence information is available.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Endogenous gene" refers to a native gene in its natural location in the genome of an organism. A "foreign-gene" refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

"Coding sequence" refers to a nucleotide sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, and polyadenylation recognition sequences.

"Promoter" refers to a nucleotide sequence capable of controlling the expression of a coding sequence or functional RNA. In general, a coding sequence is located 3' to a promoter sequence. The promoter sequence consists of proximal and more distal upstream elements, the latter elements often referred to as enhancers. Accordingly, an "enhancer" is a nucleotide sequence which can stimulate promoter activity and may be an innate element of the promoter or a heterologous element inserted to enhance the level or tissue-specificity of a promoter. Promoters may be derived in their entirety from a native gene, or may be composed of different elements derived from different promoters found in nature, or may even comprise synthetic nucleotide segments. It is understood by those skilled in the art that different promoters may direct the expression of a gene in different tissues or cell types, or at different stages of development, or in response to different environmental conditions. Promoters which cause a nucleic acid fragment to be expressed in most cell types at most times are commonly referred to as "constitutive promoters". New promoters of various types useful in plant cells are constantly being discovered; numerous examples may be found in the compilation by Okamuro and Goldberg (1989) *Biochemistry of Plants 15*:1-82*.* It is further recognized that since in most cases the exact boundaries of regulatory sequences have not been completely defined, nucleic acid fragments of different lengths may have identical promoter activity.

"Translation leader sequence" refers to a nucleotide sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner and Foster (1995) *Mol. Biotechnol. 3*:225-236).

"3' non-coding sequences" refer to nucleotide sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al. (1989) *Plant Cell 1*:671-680.

"RNA transcript" refers to the product resulting from RNA polymerase-catalyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA (mRNA)" refers to the RNA that is without introns and that can be translated into polypeptides by the cell. "cDNA" refers to DNA that is complementary to and derived from an mRNA template. The cDNA can be single-stranded or converted to double stranded form using, for example, the Klenow fragment of DNA polymerase I. "Sense-RNA" refers to an RNA transcript that includes the mRNA and so can be translated into a polypeptide by the cell. "Antisense RNA" refers to an RNA transcript that is complementary to all or part of a target primary transcript or mRNA and that blocks the expression of a target gene (see U.S. Patent No. 5,107,065, incorporated herein by reference). The complementarity of an antisense RNA may be with any part of the specific nucleotide sequence, i.e., at the 5' non-coding sequence, 3' non-coding sequence, introns, or the coding sequence. "Functional RNA" refers to sense RNA, antisense RNA, ribozyme RNA, or other RNA that may not be translated but yet has an effect on cellular processes.The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter). Coding sequences can be operably linked to regulatory sequences in a sense or antisense orientation. In another example, the complementary RNA regions of the invention can be operably linked, either directly or indirectly, 5' to the target mRNA, or 3' to the target mRNA, or within the target mRNA, or a first complementary region is 5' and its complement is 3' to the target mRNA.

The term "expression", as used herein, refers to the transcription and stable accumulation of sense (mRNA) or antisense RNA derived from the nucleic acid fragment of the invention. Expression may also refer to translation of mRNA into a polypeptide. "Antisense inhibition" refers to the production of antisense RNA transcripts capable of suppressing the expression of the target protein. "Overexpression" refers to the production of a gene product in transgenic organisms that exceeds levels of production in normal or non-transformed organisms. "Co-suppression" refers to the production of sense RNA transcripts capable of suppressing the expression of identical or substantially similar foreign or endogenous genes (U.S. Patent No. 5,231,020).

A "protein" or "polypeptide" is a chain of amino acids arranged in a specific order determined by the coding sequence in a polynucleotide encoding the polypeptide. Each protein or polypeptide has a unique function.

"Altered levels" or "altered expression" refers to the production of gene product(s) in transgenic organisms in amounts or proportions that differ from that of normal or non-transformed organisms.

"Mature protein" or the term "mature" when used in describing a protein refers to a post-translationally processed polypeptide; i.e., one from which any pre- or propeptides present in the primary translation product have been removed. "Precursor protein" or the term "precursor" when used in describing a protein refers to the primary product of translation of mRNA; i.e-, with pre- and propeptides still present. Pre- and propeptides may be but are not limited to intracellular localization signals.

A "chloroplast transit peptide" is an amino acid sequence which is translated in conjunction with a protein and directs the protein to the chloroplast or other plastid types present in the cell in which the protein is made. "Chloroplast transit sequence" refers to a nucleotide sequence that encodes a chloroplast transit peptide. A "signal peptide" is an amino acid sequence which is translated in conjunction with a protein and directs the protein to the secretory system (Chrispeels (1991) *Ann. Rev Plant Phys. Plant Mol. Biol. 42*:21-53). If the protein is to be directed to a vacuole, a vacuolar targeting signal (*supra*) can further be added, or if to the endoplasmic reticulum, an endoplasmic reticulum retention signal (*supra*) may be added. If the protein is to be directed to the nucleus, any signal peptide present should be removed and instead a nuclear localization signal included (Raikhel (1992) *Plant Phys. 100:*1627-1632).

"Transformation" refers to the transfer of a nucleic acid fragment into the genome of a host organism, resulting in genetically stable inheritance. Host organisms containing the transformed nucleic acid fragments are referred to as "transgenic" organisms. Examples of methods of plant transformation include *Agrobacterium-*mediated transformation (De Blaere et al. (1987) *Meth. Enzymol. 143*:277) and particle-accelerated or "gene gun" transformation technology (Klein et al. (1987) *Nature (London) 32*7:70-73; U.S. Patent No. 4,945,050). Thus, isolated polynucleotides of the present invention can be incorporated into recombinant constructs, typically DNA constructs, capable of introduction into and replication in a host cell. Such a construct can be a vector that includes a replication system and sequences that are capable of transcription and translation of a polypeptide-encoding sequence in a given host cell. A number of vectors suitable for stable transfection of plant cells or for the establishment of transgenic plants have been described in, e.g., Pouwels et al., Cloning Vectors: A Laboratory Manual, 1985, supp. 1987; Weissbach and Weissbach, Methods for Plant Molecular Biology, Academic Press, 1989; and Flevin et al., Plant Molecular Biology Manual, Kluwer Academic Publishers, 1990. Typically, plant expression vectors include, for example, one or more cloned plant genes under the transcriptional control of 5' and 3' regulatory sequences and a dominant selectable marker. Such plant expression vectors also can contain a promoter regulatory region (e.g., a regulatory region controlling inducible or constitutive, environmentally- or developmentally-regulated, or cell- or tissue-specific expression), a transcription initiation start site, a ribosome binding site, an RNA processing signal, a transcription termination site, and/or a polyadenylation signal.Standard recombinant DNA and molecular cloning techniques used herein are well known in the art and are described more fully in Sambrook et al. *Molecular Cloning: A Laboratory Manual;* Cold Spring Harbor Laboratory Press: Cold Spring Harbor, 1989 (hereinafter "Maniatis").

"PCR"' or "polymerase chain reaction" is well known by those skilled in the art as a technique used for the amplification of specific DNA segments (U.S. Patent Nos. 4,683,195 and 4,800,159).

The terms "recombinant construct", "expression construct", recombinant expression construct", "chimeric construct" and "chimeric gene." are used interchangeably herein. Such construct may be itself or may be used in conjunction with a vector. If a vector is used then the choice of vector is dependent upon the method that will be used to transform host plants as is well known to those skilled in the art. For example, a plasmid vector can be used. The skilled artisan is well aware of the genetic elements that must be present on the vector in order to successfully transform, select and propagate host cells comprising any of the isolated nucleic acid fragments of the invention. The skilled artisan will also recognize that different independent transformation events will result in different levels and patterns of expression (Jones et al, (1985) *EMBO J. 4*:2411-2418; De Almeida et al, (1989) *Mol. Gen. Genetics 218*:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

The present invention concerns an isolated polynucleotide comprising a nucleotide sequence selected from the group consisting of: (a) first nucleotide sequence encoding a polypeptide which is a cytochrome P450 enzyme associated with the synthesis of delta 12-epoxy fatty acids wherein said polypeptide has at least 50%, 55%, 60%, 65%, 70%, 75%, 80%, 85%, 90%, or 95% identity based on the Clustal method of alignment when compared to a polypeptide of SEQ ID NO:2, or (b) a second nucleotide sequence comprising the complement of the first nucleotide sequence.

Preferably, the first nucleotide sequence comprises a nucleic acid sequence selected from the group consisting of SEQ ID NO:1, that codes for the polypeptide of SEQ ID NO:2.

The present invention suggests that the catalytic activity responsible for epoxy group formation in *Euphorbia lagascae* is an enzyme of the cytochrome P450 mono-oxygenase class. It is believed that none of the genes encoding this type of activity has been characterized, prior to this application. Epoxygenase activities from other plants producing vernolic acid have been shown to be different from the *Euphorbia lagascae* enzyme. Genes from *Vernonia galamensis* and *Crepis palaestina* have been isolated, and when expressed in plants or yeast, the encoded proteins are capable of converting linoleic acid to vernolic acid (Lee et al. (1998) *Science* 280:915-918, and U.S. Patent No. 5,846,784). Unlike the *Euphorbia lagascae* enzyme, these enzymes are not cytochrome P450-dependent but are instead related to the endoplasmic reticulum-localized fatty acid desaturases (PCT Publication No. WO94/11516, published on May 26, 1994). Apparently these fatty acid epoxidizing enzymes are related in sequence to the class of membrane bound enzymes responsible for fatty acid desaturation and fatty acid hydroxylation (Broun, P. and Somerville, C. (1997) *Plant Physiol 113*: 933-942). Therefore, there are two distinct classes of genes encoding enzymes capable of forming the Δ¹²-epoxy group of vemolic acid: one that is cytochrome P450-dependent and the other that is related to the fatty acid desaturases and hydroxylases.

Clones for three different cytochrome P450 enzymes were identified among approximately 1000 total expressed sequence tags (ESTs) generated from a *E. lagascae* developing seed cDNA library. One of the clones, eellc.pk002.i4, was expressed behind a *GAL1* promoter in a yeast (*Saccharomyces cerevisiae*) strain that contains a plant cytochrome P450 reductase integrated into its genome. The cells were provided with exogenous linoleic acid (18:2) which is believed to be the fatty acid substrate for vemolic acid synthesis. In cultures maintained at 28°C, vemolic acid was detected in amounts of 1 to 5 wt% of the total fatty acid. This fatty acid is not normally detected in *Saccharomyces cerevisiae.* The presence of vernolic acid in cells expressing eel1c.pk002.i4 was confirmed by GC-MS analysis of fatty acid methyl esters prepared by either basic (sodium methoxide) or acidic (sulfuric acid/methanol) transesterification procedures. This finding is consistent with the involvement of a cytochrome P450 enzyme (corresponding to eellc.pk002.i4) in vernolic acid synthesis in *Euphorbia lagascae* seeds.

This is believed to be the first characterization of a plant gene encoding a cytochrome P450-class enzyme capable of producing Δ¹²-epoxidized fatty acids.

Nucleic acid fragments encoding at least a portion of several cytochrome P450 enzymes associated with the synthesis of plant Δ¹²-epoxy fatty acids have been isolated and identified by comparison of random plant cDNA sequences to public databases containing nucleotide and protein sequences using the BLAST algorithms well known to those skilled in the art. The nucleic acid fragments of the instant invention may be used to isolate cDNAs and genes encoding homologous proteins from the same or other plant species. Isolation of homologous genes using sequence-dependent protocols is well known in the art. Examples of sequence-dependent protocols include, but are not limited to, methods of nucleic acid hybridization, and methods of DNA and RNA amplification as exemplified by various uses of nucleic acid amplification technologies (e.g., polymerase chain reaction, ligase chain reaction).

For example, genes encoding other plant epoxygenase, either as cDNAs or genomic DNAs, could be isolated directly by using all or a portion of the instant nucleic acid fragments as DNA hybridization probes to screen libraries from any desired plant employing methodology well known to those skilled in the art. Specific oligonucleotide probes based upon the instant nucleic acid sequences can be designed and synthesized by methods known in the art (Maniatis). Moreover, an entire sequence can be used directly to synthesize DNA probes by methods known to the skilled artisan such as random primer DNA labeling, nick translation, end-labeling techniques, or RNA probes using available *in vitro* transcription systems. In addition, specific primers can be designed and used to amplify a part or all of the instant sequences. The resulting amplification products can be labeled directly during amplification reactions or labeled after amplification reactions, and used as probes to isolate full length cDNA or genomic fragments under conditions of appropriate stringency.

In addition, two short segments of the instant nucleic acid fragments may be used in polymerase chain reaction protocols to amplify longer nucleic acid fragments encoding homologous genes from DNA or RNA. The polymerase chain reaction may also be performed on a library of cloned nucleic acid fragments wherein the sequence of one primer is derived from the instant nucleic acid fragments, and the sequence of the other primer takes advantage of the presence of the polyadenylic acid tracts to the 3' end of the mRNA precursor encoding plant genes. Alternatively, the second primer sequence may be based upon sequences derived from the cloning vector. For example, the skilled artisan can follow the RACE protocol (Frohman et al. (1988) *Proc. Natl. Acad. Sci. USA* 85:8998-9002) to generate cDNAs by using PCR to amplify copies of the region between a single point in the transcript and the 3' or 5' end. Primers oriented in the 3' and 5' directions can be designed from the instant sequences. Using commercially available 3' RACE or 5' RACE systems (BRL), specific 3' or 5' cDNA fragments can be isolated (Ohara et al. (1989) *Proc. Natl. Acad. Sci. USA* 86:5673-5677; Loh et al. (1989) *Science* 243:217-220). Products generated by the 3' and 5' RACE procedures can be combined to generate full-length cDNAs (Frohman and Martin (1989) *Techniques 1*:165). Consequently, a polynucleotide comprising a nucleotide sequence of at least one of 60 (preferably one of at least 40, most preferably one of at least 30) contiguous nucleotides derived from a nucleotide sequence of SEQ ID NO:1, and the complement of such nucleotide sequences may be used in such methods to obtain a nucleic acid fragment encoding a substantial portion of an amino acid sequence of a polypeptide.

The specification discloses a method of obtaining a nucleic acid fragment encoding a substantial portion of a plant epoxygenase polypeptide, preferably a substantial portion of a plant epoxygenase polypeptide, comprising the steps of: synthesizing an oligonucleotide primer comprising a nucleotide sequence of at least one of 60 (preferably at least one of 40, most preferably at least one of 30) contiguous nucleotides derived from a nucleotide sequence of SEQ ID NO:1, and the complement of such nucleotide sequences; and amplifying a nucleic acid fragment (preferably a cDNA inserted in a cloning vector) using the oligonucleotide primer. The amplified nucleic acid fragment preferably will encode a portion of a plant epoxygenase polypeptide.

Availability of the instant nucleotide and deduced amino acid sequences facilitates immunological screening of cDNA expression libraries. Synthetic peptides representing portions of the instant amino acid sequences may be synthesized. These peptides can be used to immunize animals to produce polyclonal or monoclonal antibodies with specificity for peptides or proteins comprising the amino acid sequences. These antibodies can be then be used to screen cDNA expression libraries to isolate full-length cDNA clones of interest (Lerner (1984) *Adv. Immunol. 36*:1-34; Maniatis).

In another embodiment, this invention concerns host cells comprising either the chimeric genes of the invention as described herein or an isolated polynucleotide of the invention as described herein. Examples of host cells which can be used to practice the invention include, but are not limited to, yeast, bacteria, and plants.

As was noted above, the nucleic acid fragments of the instant invention may be used to create transgenic plants in which the disclosed polypeptides are present at higher or lower levels than normal or in cell types or developmental stages in which they are not normally found. This would have the effect of altering the level of fatty acids containing epoxide groups in those cells.

Overexpression of the proteins of the instant invention may be accomplished by first constructing a chimeric gene in which the coding region is operably linked to a promoter capable of directing expression of a gene in the desired tissues at the desired stage of development The chimeric gene may comprise promoter sequences and translation leader sequences derived from the same genes 3' Non-coding sequences encoding transcription termination signals may also be provided. The instant chimeric gene may also comprise one or more introns in order to facilitate gene expression.

Plasmid vectors comprising the instant isolated polynucleotide (or chimeric gene) may be constructed. The choice of plasmid vector is dependent upon the method that will be used to transform host plants. The skilled artisan is well aware of the genetic elements that must be present on the plasmid vector in order to successfully transform, select and propagate host cells containing the chimeric gene. The skilled artisan will also recognize that different independent transformation events will result in different levels and patterns of expression (Jones et al. (1985) *EMBO J. 4*:2411-2418; De Almeida et al. (1989) *Mol. Gen. Genetics 218*:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

For some applications it may be useful to direct the instant polypeptide to different cellular compartments, or to facilitate its secretion from the cell. It is thus envisioned that the chimeric gene described above may be further supplemented by directing the coding sequence to encode the instant polypeptide with appropriate intracellular targeting sequences such as transit sequences (Keegstra (1989) *Cell 56*:247-253), signal sequences or sequences encoding endoplasmic reticulum localization (Chrispeels (1991) *Ann. Rev. Plant Phys. Plant Mol. Biol. 42*:21-53), or nuclear localization signals (Raikhel (1992) *Plant Phys.100*:1627-1632) with or without removing targeting sequences that are already present. While the references cited give examples of each of these, the list is not exhaustive and more targeting signals of use may be discovered in the future.

It may also be desirable to reduce or eliminate expression of genes encoding the instant polypeptides in plants for some applications. In order to accomplish this, a chimeric gene designed for co-suppression of the instant polypeptide can be constructed by linking a gene or gene fragment encoding that polypeptide to plant promoter sequences. Alternatively, a chimeric gene designed to express antisense RNA for all or part of the instant nucleic acid fragment can be constructed by linking the gene or gene fragment in reverse orientation to plant promoter sequences. Either the co-suppression or antisense chimeric genes could be introduced into plants via transformation wherein expression of the corresponding endogenous genes are reduced or eliminated.

Molecular genetic solutions to the generation of plants with altered gene expression have a decided advantage over more traditional plant breeding approaches. Changes in plant phenotypes can be produced by specifically inhibiting expression of one or more genes by antisense inhibition or cosuppression (U.S. Patent Nos. 5,190,931, 5,107,065 and 5,283,323). An antisense or cosuppression construct would act as a dominant negative regulator of gene activity. While conventional mutations can yield negative regulation of gene activity these effects are most likely recessive. The dominant negative regulation available with a transgenic approach may be advantageous from a breeding perspective. In addition, the ability to restrict the expression of a specific phenotype to the reproductive tissues of the plant by the use of tissue specific promoters may confer agronomic advantages relative to conventional mutations which may have an effect in all tissues in which a mutant gene is ordinarily expressed.

The person skilled in the art will know that special considerations are associated with the use of antisense or cosuppression technologies in order to reduce expression of particular genes. For example, the proper level of expression of sense or antisense genes may require the use of different chimeric genes utilizing different regulatory elements known to the skilled artisan. Once transgenic plants are obtained by one of the methods described above, it will be necessary to screen individual transgenics for those that most effectively display the desired phenotype. Accordingly, the skilled artisan will develop methods for screening large numbers of transformants. The nature of these screens will generally be chosen on practical grounds. For example, one can screen by looking for changes in gene expression by using antibodies specific for the protein encoded by the gene being suppressed, or one could establish assays that specifically measure enzyme activity. A preferred method will be one which allows large numbers of samples to be processed rapidly, since it will be expected that a large number of transformants will be negative for the desired phenotype.

A polypeptide (or portions thereof) may be produced in heterologous host cells, particularly in the cells of microbial hosts, and can be used to prepare antibodies to this protein by methods well known to those skilled in the art. The antibodies are useful for detecting the polypeptide of the instant invention *in situ* in cells or *in vitro* in cell extracts. Preferred heterologous host cells for production of the instant polypeptide are microbial hosts. Microbial expression systems and expression vectors containing regulatory sequences that direct high level expression of foreign proteins are well known to those skilled in the art. Any of these could be used to construct a chimeric gene for production of a polypeptide. This chimeric gene could then be introduced into appropriate microorganisms via transformation to provide high level expression of the encoded cytochrome P450 enzymes associated with the synthesis of plant Δ¹²-epoxy fatty acids. An example of a vector for high level expression of the instant polypeptide in a bacterial host is provided (Example 6).

All or a substantial portion of the polynucleotides of the instant invention may also be used as probes for genetically and physically mapping the genes that they are a part of, and used as markers for traits linked to those genes. Such information may be useful in plant breeding in order to develop lines with desired phenotypes. For example, the instant nucleic acid fragments may be used as restriction fragment length polymorphism (RFLP) markers. Southern blots (Maniatis) of restriction-digested plant genomic DNA may be probed with the nucleic acid fragments of the instant invention. The resulting banding patterns may then be subjected to genetic analyses using computer programs such as MapMaker (Lander et al. (1987) *Genomics 1*:174-181) in order to construct a genetic map. In addition, the nucleic acid fragments of the instant invention may be used to probe Southern blots containing restriction endonuclease-treated genomic DNAs of a set of individuals representing parent and progeny of a defined genetic cross. Segregation of the DNA polymorphisms is noted and used to calculate the position of the instant nucleic acid sequence in the genetic map previously obtained using this population (Botstein et al. (1980) *Am. J. Hum. Genet. 32*:314-331).

The production and use of plant gene-derived probes for use in genetic mapping is described in Bernatzky and Tanksley (1986) *Plant Mol. Biol. Reporter 4*:37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

Nucleic acid probes derived from the instant nucleic acid sequences may also be used for physical mapping (i.e., placement of sequences on physical maps; *see* Hoheisel et al. In: *Nonmammalian Genomic Analysis: A Practical Guide,* Academic press 1996, pp. 319-346, and references cited therein).

Nucleic acid probes derived from the instant nucleic acid sequences may be used in direct fluorescence *in situ* hybridization (FISH) mapping (Trask (1991) *Trends Genet. 7*:149-154). Although current methods of FISH mapping favor use of large clones (several to several hundred KB; see Laan et al. (1995) *Genome Res 5:*13-20), improvements in sensitivity may allow performance of FISH mapping using shorter probes.

A variety of nucleic acid amplification-based methods of genetic and physical mapping may be carried out using the instant nucleic acid sequences. Examples include allele-specific amplification (Kazazian (1989) *J. Lab. Clin. Med. 11*:95-96), polymorphism of PCR-amplified fragments (CAPS; Sheffield et al. (1993) *Genomics 16*:325-332), allele-specific ligation (Landegren et al. (1988) *Science 241*:1077-1080), nucleotide extension reactions (Sokolov (1990) *Nucleic Acid Res. 18*:3671), Radiation Hybrid Mapping (Walter et al. (1997) *Nat. Gener. 7*:22-28) and Happy Mapping (Dear and Cook (1989) *Nucleic Acid Res. 17:*6795-6807*).* For these methods, the sequence of a nucleic acid fragment is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

In a further embodiment, the present invention concerns a method of selecting an isolated polynucleotide that affects the level of Δ¹² epoxy fatty acids in a suitable host cell, the method comprising the steps of, (a) making an isolated polynucleotide comprising the polynucleotide sequence of the present invention, (b) introducing the isolated polynucleotide into a suitable host cell and (c) determining presence or absence of Δ¹² epoxy fatty acids in the host cell of (b).

In yet another embodiment, the present invention relates to a method for producing Δ¹² epoxy fatty acids in a suitable host cell which comprises, (a) transforming a suitable host cell with the chimeric construct of the present invention, (b) growing the transformed host cells of step (a), and (c) determining the presence or absence of Δ¹² epoxy fatty acids in the transformed cells of (b).

### EXAMPLES

The present invention is further defined in the following Examples, in which parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions. Thus, various modifications of the invention in addition to those shown and described herein will be apparent to those skilled in the art from the foregoing description.

### EXAMPLE 1

### Composition of cDNA Libraries; Isolation and Sequencing of cDNA Clones

A cDNA library representing mRNAs from *Euphorbia lagascae* seeds was prepared. The characteristics of the library are described below.

**TABLE 2**

| cDNA Library from *Euphorbia lagascae* | | |
|---|---|---|
| Library | Tissue | Clone |
| eellc | *Euphorbia lagascae* developing seeds which could contain a putative cytochrome P450 involved in epoxy fatty acid synthesis | eellc.pk002.i4 |

cDNA libraries may be prepared by any one of many methods available. For example, the cDNAs may be introduced into plasmid vectors by first preparing the cDNA libraries in Uni-ZAP^{™} XR vectors according to the manufacturer's protocol (Stratagene Cloning Systems, La Jolla, CA). The Uni-ZAP^{™} XR libraries are converted into plasmid libraries according to the protocol provided by Stratagene. Upon conversion, cDNA inserts will be contained in the plasmid vector pBluescript. In addition, the cDNAs may be introduced directly into precut Bluescript II SK(+) vectors (Stratagene) using T4 DNA ligase (New England Biolabs), followed by transfection into DH10B cells according to the manufacturer's protocol (GIBCO BRL Products). Once the cDNA inserts are in plasmid vectors, plasmid DNAs are prepared from randomly picked bacterial colonies containing recombinant pBluescript plasmids, or the insert cDNA sequences are amplified via polymerase chain reaction using primers specific for vector sequences flanking the inserted cDNA sequences. Amplified insert DNAs or plasmid DNAs are sequenced in dye-primer sequencing reactions to generate partial cDNA sequences (expressed sequence tags or "ESTs"; see Adams et al., (1991) *Science* 252:1651-1656). The resulting ESTs are analyzed using a Perkin Elmer Model 377 fluorescent sequencer.

Full-insert sequence (FIS) data is generated utilizing a modified transposition protocol. Clones identified for FIS are recovered from archived glycerol stocks as single colonies, and plasmid DNAs are isolated via alkaline lysis. Isolated DNA templates are reacted with vector primed M13 forward and reverse oligonucleotides in a PCR-based sequencing reaction and loaded onto automated sequencers. Confirmation of clone identification is performed by sequence alignment to the original EST sequence from which the FIS request is made.

Confirmed templates are transposed via the Primer Island transposition kit (PE Applied Biosystems, Foster City, CA) which is based upon the *Saccharomyces cerevisiae* Tyl transposable element (Devine and Boeke (1994) *Nucleic Acids Res. 22*:3765-3772). The *in vitro* transposition system places unique binding sites randomly throughout a population of large DNA molecules. The transposed DNA is then used to transform DH10B electro-competent cells (Gibco BRL/Life Technologies, Rockville, MD) via electroporation. The transposable element contains an additional selectable marker (named DHFR; Fling and Richards (1983) *Nucleic Acids Res. 11*:5147-5158), allowing for dual selection on agar plates of only those subclones containing the integrated transposon. Multiple subclones are randomly selected from each transposition reaction, plasmid DNAs are prepared via alkaline lysis, and templates are sequenced (ABI Prism dye-terminator ReadyReaction mix) outward from the transposition event site, utilizing unique primers specific to the binding sites within the transposon.

Sequence data is collected (ABI Prism Collections) and assembled using Phred/Phrap (P. Green, University of Washington, Seattle). Phred/Phrap is a public domain software program which re-reads the ABI sequence data, re-calls the bases, assigns quality values, and writes the base calls and quality values into editable output files. The Phrap sequence assembly program uses these quality values to increase the accuracy of the assembled sequence contigs. Assemblies are viewed by the Consed sequence editor (D. Gordon, University of Washington, Seattle).

### EXAMPLE 2

### Identification of cDNA Clones

cDNA clones encoding cytochrome P450 enzymes associated with the synthesis of of plant Δ¹²-epoxy fatty acids were identified by conducting BLAST (Basic Local Alignment Search Tool; Altschul et al. (1993) *J. Mol. Biol. 215*:403-410; see also www.ncbi.nlm.nih.gov/BLAST/) searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences obtained in Example 1 were analyzed for similarity to all publicly available DNA sequences contained in the "nr" database using the BLASTN algorithm provided by the National Center for Biotechnology Information (NCBI). The DNA sequences were translated in all reading frames and compared for similarity to all publicly available protein sequences contained in the "nr" database using the BLASTX algorithm (Gish and States (1993) *Nat. Genet. 3*:266-272) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the cDNA sequence and the BLAST "hit" represent homologous proteins.

ESTs submitted for analysis are compared to the genbank database as described above. ESTs that contain sequences more 5- or 3-prime can be found by using the BLASTn algorithm (Altschul et al (1997) *Nucleic Acids Res. 25*:3389-3402.) against the Du Pont proprietary database comparing nucleotide sequences that share common or overlapping regions of sequence homology. Where common or overlapping sequences exist between two or more nucleic acid fragments, the sequences can be assembled into a single contiguous nucleotide sequence, thus extending the original fragment in either the 5- or 3-prime direction. Once the most 5-prime EST is identified, its complete sequence can be determined by Full Insert Sequencing as described in Example 1. Homologous genes belonging to different species can be found by comparing the amino acid sequence of a known gene (from either a proprietary source or a public database) against an EST database using the tBLASTn algorithm. The tBLASTn algorithm searches an amino acid query against a nucleotide database that is translated in all 6 reading frames. This search allows for differences in nucleotide codon usage between different species, and for codon degeneracy.

### EXAMPLE 3

### Characterization of cDNA Clones Encoding Plant Epoxygenases

The BLASTX search using the EST sequences from clones listed in Table 3 revealed similarity of the polypeptides encoded by the cDNAs to a cytochrome P450 heme-containing polypeptide involved in fungal incompatibility interactions from pepper (*Capsicum annuum* NCBI General Identifier No. gi 6739506). Shown in Table 3 are the BLAST results for the sequence of the entire cDNA insert comprising the indicated cDNA clone ("FIS"), that also comprises the complete gene sequence for a *Euphorbia* epoxygenase:

**TABLE 3**

| BLAST Results for Sequences Encoding Polypeptides With Plant Epoxygenase Activity | | |
|---|---|---|
| | | BLAST pLog Score |
| Clone | Status | 6739506 |
| eel 1 c.pk002.i4 | FIS | 122.00 |

Figure 1 presents an alignment of the amino acid sequence set forth in SEQ ID NO:2 and the pepper sequence (SEQ ID NO:3). The data in Table 4 represents a calculation of the percent identity of the amino acid sequences set forth in SEQ ID NO:2 and the pepper (*Capsicum annuum*) sequence (SEQ ID NO:3).

**TABLE 4**

| Percent Identity of Amino Acid Sequences Deduced From the Nucleotide Sequences of cDNA Clones Encoding Polypeptides With Plant Epoxygenase Activity | |
|---|---|
| SEQ ID NO. | Percent Identity to 6739506 |
| 2 | 40.4% |

Sequence alignments and percent identity calculations were performed using the Megalign program of the LASERGENE bioinformatics computing suite (DNASTAR Inc., Madison, WI). Multiple alignment of the sequences was performed using the Clustal method of alignment (Higgins and Sharp (1989) *CABIOS. 5:*151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5. Sequence alignments and BLAST scores and probabilities indicate that the nucleic acid fragments comprising the instant cDNA clones encode a substantial portion of a plant epoxygenase. These sequences represent the first cytochrome P450 sequences encoding an enzyme associated with Δ¹² epoxy group formation in plants known to Applicant.

### EXAMPLE 4

### Expression of Chimeric Genes in Monocot Cells

A chimeric gene comprising a cDNA encoding the instant polypeptide in sense orientation with respect to the maize 27 kD zein promoter that is located 5' to the cDNA fragment, and the 10 kD zein 3' end that is located 3' to the cDNA fragment, can be constructed. The cDNA fragment of this gene may be generated by polymerase chain reaction (PCR) of the cDNA clone using appropriate oligonucleotide primers. Cloning sites (NcoI or SmaI) can be incorporated into the oligonucleotides to provide proper orientation of the DNA fragment when inserted into the digested vector pML103 as described below. Amplification is then performed in a standard PCR. The amplified DNA is then digested with restriction enzymes NcoI and SmaI and fractionated on an agarose gel. The appropriate band can be isolated from the gel and combined with a 4.9 kb NcoI-SmaI fragment of the plasmid pML103. Plasmid pML103 has been deposited under the terms of the Budapest Treaty at ATCC (American Type Culture Collection, 10801 University Blvd., Manassas, VA 20110-2209), and bears accession number ATCC 97366. The DNA segment from pML103 contains a 1.05 kb Sall-NcoI promoter fragment of the maize 27 kD zein gene and a 0.96 kb SmaI-SalI fragment from the 3' end of the maize 10 kD zein gene in the vector pGem9Zf(+) (Promega). Vector and insert DNA can be ligated at 15°C overnight, essentially as described (Maniatis). The ligated DNA may then be used to transform *E. coli* XL1-Blue (Epicurian Coli XL-1 Blue^{™}; Stratagene). Bacterial transformants can be screened by restriction enzyme digestion of plasmid DNA and limited nucleotide sequence analysis using the dideoxy chain termination method (Sequenase^{™} DNA Sequencing Kit; U.S. Biochemical). The resulting plasmid construct would comprise a chimeric gene encoding, in the 5' to 3' direction, the maize 27 kD zein promoter, a cDNA fragment encoding the instant polypeptide, and the 10 kD zein 3' region.

The chimeric gene described above can then be introduced into corn cells by the following procedure. Immature corn embryos can be dissected from developing caryopses derived from crosses of the inbred corn lines H99 and LH132. The embryos are isolated 10 to 11 days after pollination when they are 1.0 to 1.5 mm long. The embryos are then placed with the axis-side facing down and in contact with agarose-solidified N6 medium (Chu et al. (1975) *Sci. Sin. Peking* 18:659-668). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and sub-cultured on this medium every 2 to 3 weeks.

The plasmid, p35S/Ac (obtained from Dr. Peter Eckes, Hoechst Ag, Frankfurt, Germany) may be used in transformation experiments in order to provide for a selectable marker. This plasmid contains the *Pat* gene (see European Patent Publication 0 242 236) which encodes phosphinothricin acetyl transferase (PAT). The enzyme PAT confers resistance to herbicidal glutamine synthetase inhibitors such as phosphinothricin. The *pat* gene in p35S/Ac is under the control of the 35S promoter from Cauliflower Mosaic Virus (Odell et al. (1985) *Nature* 313:810-812) and the 3' region of the nopaline synthase gene from the T-DNA of the Ti plasmid of *Agrobacterium tumefaciens.*

The particle bombardment method (Klein et al. (1987) *Nature* 327:70-73) may be used to transfer genes to the callus culture cells. According to this method, gold particles (1 µm in diameter) are coated with DNA using the following technique. Ten µg of plasmid DNAs are added to 50 µL of a suspension of gold particles (60 mg per mL). Calcium chloride (50 µL of a 2.5 M solution) and spermidine free base (20 µL of a 1.0 M solution) are added to the particles. The suspension is vortexed during the addition of these solutions. After 10 minutes, the tubes are briefly centrifuged (5 sec at 15,000 rpm) and the supernatant removed. The particles are resuspended in 200 µL of absolute ethanol, centrifuged again and the supernatant removed. The ethanol rinse is performed again and the particles resuspended in a final volume of 30 µL of ethanol. An aliquot (5 µL) of the DNA-coated gold particles can be placed in the center of a Kapton^{™} flying disc (Bio-Rad Labs). The particles are then accelerated into the corn tissue with a Biolistic^{™} PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains gluphosinate (2 mg per liter) and lacks casein or proline. The tissue continues to grow slowly on this medium. After an additional 2 weeks the tissue can be transferred to fresh N6 medium containing gluphosinate. After 6 weeks, areas of about 1 cm in diameter of actively growing callus can be identified on some of the plates containing the glufosinate-supplemented medium. These calli may continue to grow when sub-cultured on the selective medium.

Plants can be regenerated from the transgenic callus by first transferring clusters of tissue to N6 medium supplemented with 0.2 mg per liter of 2,4-D. After two weeks the tissue can be transferred to regeneration medium (Fromm et al. (1990) *Bio*/*Technology 8*:833-839).

### EXAMPLE 5

### Expression of Chimeric Genes in Dicot Cells

A seed-specific expression cassette composed of the promoter and transcription terminator from the gene encoding the β subunit of the seed storage protein phaseolin from the bean *Phaseolus vulgaris* (Doyle et al. (1986) *J. Biol. Chem. 261*:9228-9238) can be used for expression of the instant polypeptide in transformed soybean. The phaseolin cassette includes about 500 nucleotides upstream (5') from the translation initiation codon and about 1650 nucleotides downstream (3') from the translation stop codon of phaseolin. Between the 5' and 3' regions are the unique restriction endonuclease sites Nco I (which includes the ATG translation initiation codon), Sma I, Kpn I and Xba I. The entire cassette is flanked by Hind III sites.

The cDNA fragment of this gene may be generated by polymerase chain reaction (PCR) of the cDNA clone using appropriate oligonucleotide primers. Cloning sites can be incorporated into the oligonucleotides to provide proper orientation of the DNA fragment when inserted into the expression vector. Amplification is then performed as described above, and the isolated fragment is inserted into a pUC 18 vector carrying the seed expression cassette.

Soybean embryos may then be transformed with the expression vector comprising sequences encoding the instant polypeptide. To induce somatic embryos, cotyledons, 3-5 mm in length dissected from surface sterilized, immature seeds of the soybean cultivar A2872, can be cultured in the light or dark at 26°C on an appropriate agar medium for 6-10 weeks. Somatic embryos which produce secondary embryos are then excised and placed into a suitable liquid medium. After repeated selection for clusters of somatic embryos which multiplied as early, globular staged embryos, the suspensions are maintained as described below.

Soybean embryogenic suspension cultures can be maintained in 35 mL liquid media on a rotary shaker, 150 rpm, at 26°C with florescent lights on a 16:8 hour day/night schedule. Cultures are subcultured every two weeks by inoculating approximately 35 mg of tissue into 35 mL of liquid medium.

Soybean embryogenic suspension cultures may then be transformed by the method of particle gun bombardment (Klein et al. (1987) *Nature* (London) *327*:70-73, U.S. Patent No. 4,945,050). A DuPont Biolistic^{™} PDS 1000/HE instrument (helium retrofit) can be used for these transformations.

A selectable marker gene which can be used to facilitate soybean transformation is a chimeric gene composed of the 35S promoter from Cauliflower Mosaic Virus (Odell et al. (1985) *Nature 313*:810-812), the hygromycin phosphotransferase gene from plasmid pJR225 (from *E. coli*; Gritz et al.(1983) *Gene 25*:179-188) and the 3' region of the nopaline synthase gene from the T-DNA of the Ti plasmid of *Agrobacterium tumefaciens.* The seed expression cassette comprising the phaseolin 5' region, the fragment encoding the instant polypeptide and the phaseolin 3' region can be isolated as a restriction fragment. This fragment can then be inserted into a unique restriction site of the vector carrying the marker gene.

To 50 µL of a 60 mg/mL 1 µm gold particle suspension is added (in order): 5 µL DNA (1 µg/µL), 20 µl spermidine (0.1 M), and 50 µL CaCl₂ (2.5 M). The particle preparation is then agitated for three minutes, spun in a microfuge for 10 seconds and the supernatant removed. The DNA-coated particles are then washed once in 400 µL 70% ethanol and resuspended in 40 µL of anhydrous ethanol. The DNA/particle suspension can be sonicated three times for one second each. Five µL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post bombardment with fresh media containing 50 mg/mL hygromycin. This selective media can be refreshed weekly. Seven to eight weeks post bombardment, green, transformed tissue may be observed growing from untransformed, necrotic embryogenic clusters. Isolated green tissue is removed and inoculated into individual flasks to generate new, clonally própagated, transformed embryogenic suspension cultures. Each new line may be treated as an independent transformation event. These suspensions can then be subcultured and maintained as clusters of immature embryos or regenerated into whole plants by maturation and germination of individual somatic embryos.

### EXAMPLE 6

### Expression of Chimeric Genes in Microbial Cells

The cDNAs encoding the instant polypeptide can be inserted into the T7 *E. coli* expression vector pBT430. This vector is a derivative of pET-3a (Rosenberg et al. (1987) *Gene 56:*125-135) which employs the bacteriophage T7 RNA polymerase/T7 promoter system. Plasmid pBT430 was constructed by first destroying the EcoR I and Hind III sites in pET-3at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence ofpET-3aM in this region, 5'-CATATGG, was converted to 5'-CCCATGG in pBT430.

Plasmid DNA containing a cDNA may be appropriately digested to release a nucleic acid fragment encoding the protein. This fragment may then be purified on a 1% low melting agarose gel. Buffer and agarose contain 10 µg/ml ethidium bromide for visualization of the DNA fragment. The fragment can then be purified from the agarose gel by digestion with GELase^{™} (Epicentre Technologies, Madison, WI) according to the manufacturer's instructions, ethanol precipitated, dried and resuspended in 20 µL of water. Appropriate oligonucleotide adapters may be ligated to the fragment using T4 DNA ligase (New England Biolabs (NEB), Beverly, MA). The fragment containing the ligated adapters can be purified from the excess adapters using low melting agarose as described above. The vector pBT430 is digested, dephosphorylated with alkaline phosphatase (NEB) and deproteinized with phenol/chloroform as described above. The prepared vector pBT430 and fragment can then be ligated at 16°C for 15 hours followed by transformation into DH5 electrocompetent cells (GIBCO BRL). Transformants can be selected on agar plates containing LB media and 100 µg/mL ampicillin. Transformants containing the gene encoding the instant polypeptide are then screened for the correct orientation with respect to the T7 promoter by restriction enzyme analysis.

For high level expression, a plasmid clone with the cDNA insert in the correct orientation relative to the T7 promoter can be transformed into *E. coli* strain BL21(DE3) (Studier et al. (1986) *J. Mol. Biol. 189*:113-130). Cultures are grown in LB medium containing ampicillin (100 mg/L) at 25°C. At an optical density at 600 nm of approximately 1, IPTG (isopropylthio-β-galactoside, the inducer) can be added to a final concentration of 0.4 mM and incubation can be continued for 3 h at 25°. Cells are then harvested by centrifugation and re-suspended in 50 µL of 50 mM Tris-HCl at pH 8.0 containing 0.1 mM DTT and 0.2 mM phenyl methylsulfonyl fluoride. A small amount of 1 mm glass beads can be added and the mixture sonicated 3 times for about 5 seconds each time with a microprobe sonicator. The mixture is centrifuged and the protein concentration of the supernatant determined. One µg of protein from the soluble fraction of the culture can be separated by SDS-polyacrylamide gel electrophoresis. Gels can be observed for protein bands migrating at the expected molecular weight.

### EXAMPLE 7

### Expression of Euphorbia lagascae EST eellc.pk002.i4 in Saccharomyces cerevisiae

The function of the cytochrome P450 enzyme corresponding to EST eellc.pk002.i4 was assessed by expression in *Saccharomyces cerevisiae* strain WHT1 [Jung W, *et al.* (2000) *Nature Biotechnol.* 18:208-212]. The WHT cell line contains the gene for NADPH-cytochrome P450 reductase from *Helianthus tuberosum* for enhancement of recombinant cytochrome P450 activity as previously described [Jung W, *et al.* (2000) *Nature Biotechnol.* 18:208-212]. The coding sequence of EST eellc.pk002.i4 was amplified by PCR using the Advantage-GC cDNA polymerase kit (Clontech) and the primer pair 5'-TCAAGGAGAAAAAACCCCGGATCCATGGAGCAGAAAAATCTCTCTTTTCCG-3' (sense; SEQ ID NO:4) and 5'-GGCCAGTGAATTGTAATACGACTCACTATAGGGCG-3' (antisense; SEQ ID NO:5). Besides homology to the cytochrome P450 coding region, these primers share homology with the vector pRS315 [Sikorski and Heiter (1989) *Genetics* 122:19-27] that was modified as previously described [Jung *et al.* (2000) *Nature Biotechnol.* 18:208-212] by the insertion of bi-directional *GAL1*/*GAL10* promoters. The resulting amplification product was hybridized to the digested vector, and transformed into *Saccharomyces cerevisiae* strain WHT1 where the expression plasmid is formed by gap repair [Hua *et al.,* (1997) *Plasmid* 38:91-96]. Proper integration of the coding sequence of EST eellc.pk002.i4 in the expression vector was confirmed by partial DNA sequencing of the resulting plasmid. Transformed yeast cells were selected for their ability to grow in the absence of leucine.

Selected colonies were grown for 3 days at 28°C in media lacking leucine [0.17% (w/v) yeast nitrogen base without amino acids (Difco), 0.5% (w/v) ammonium sulfate, 0.01% (w/v) adenine, and 0.07% (w/v) CSM-Leu (Bio101)] supplemented with glycerol and glucose to a final concentration of 5% (v/v) and 0.5% (w/v), respectively. Cells were then washed twice in the medium described above that contained 2% (w/v) galactose in place of glycerol and glucose as the carbon source. The washed cells were then diluted to OD₆₀₀≈0.4 in media consisting of 1% (w/v) yeast extract, 2% (w/v) peptone, 2% (w/v) galactose, 0.04% (w/v) adenine, and 0.2% (w/v) Tergitol NP-40. The media was also supplemented with 0.45 mM of either linoleic acid (18:2Δ^{9cis, 12cis}) or α-linolenic acid (18:3Δ^{9cis, 12cis, 15cis}). These fatty acids were added to serve as potential *in vivo* substrates for the recombinant *E. lagascae* cytochrome P450. Cultures were maintained with shaking (250 rpm) at 28°C and grown to O.D.₆₀₀≈12. Cells from 3-ml cultures were collected by centrifugation and then dried under vacuum. Fatty acid methyl esters were subsequently prepared from the dried cell pellets by direct transesterification in 1% (w/v) sodium methoxide/methanol using methods described previously [Cahoon *et al.* (2001) *J. Biol. Chem.* 276:2637-2643]. The recovered fatty acid methyl esters were then analyzed using a Hewlett-Packard 6890 gas chromatograph fitted with an Omegawax 320 column (30 m x 0.32 mm inner diameter; Supelco). The oven temperature was programmed from 220°C (2 min hold) to 240°C at a rate of 20°C/min. Retention times of epoxy fatty acid methyl esters in samples were compared to that of the vemolic acid methyl ester prepared from *E. lagascae* seed.

To confirm the identities of any Δ¹²-epoxy fatty acids produced by recombinant yeast cells, fatty acid methyl esters prepared as described above were analyzed by mass spectrometry following acid derivitization, which provides for more diagnostic structural characterization. Acidic reaction of methyl esters of Δ¹² epoxy fatty acids (*e.g.*, vernolic acid) results in the opening of the epoxy ring and the generation of two products: (1) a Δ¹²-hydroxy/Δ¹³-methoxy derivative and (2) a Δ¹²-methoxy/Δ¹³-hydroxy derivative. Fatty acid methyl esters obtained from sodium methoxide transesterification (as described above) of *S. cerevisiae* cultures were heated at 70°C in 1 ml of 2.5% (v/v) sulfuric acid in methanol for 20 min. After cooling, 1 ml of water was added, and fatty acid methyl esters were extracted with 2 ml of hexane. The fatty acid methyl esters were then dried under nitrogen, and subsequently reacted with 0.5 ml of the silylating reagent *bis-*(trimethylsilyl)trifluoroacetamide:trimethylchlorosilane (99:1, v/v) (Supelco) at 50°C for 30 min to generate trimethylsilyl (TMS) ether derivatives of hydroxyl groups. The derivatized fatty acid methyl esters were dried under nitrogen, resuspended in hexane and then analyzed using a HP6890 gas chromatograph interfaced with a HP5973 mass selective detector (Hewlett-Packard) operating at an electron impact ionization potential of 70 eV. The epoxy fatty acid methyl ester derivatives were partially resolved using a 30 m x 0.25 mm inner diameter HP-INNOWax column (Hewlett-Packard) with the oven temperature programmed from 185°C (5 min hold) to 240°C (10 min hold) at 7.5°C/min.

Using media supplemented with linoleic acid, a novel fatty acid was detected in S. *cerevisiae* WHT cells expressing the *E. lagascae* cytochrome P450 corresponding to EST eellc.pk002.i4. The methyl ester of this fatty acid is indicated as "Epoxy1" in Fig. 1B. This fatty was not detected in cells containing only the expression vector and grown under similar conditions [Figure 1A]. "Epoxy1" displayed the same retention time as the methyl ester of vernolic acid [Figure 1E]. In addition, acidic methanol derivatives of this fatty acid methyl ester displayed mass spectra [Figure 2C-D] identical to those of similarly prepared derivatives of methyl vernolic acid [Figure 2A-B]. Based on these data, "Epoxy1" in Fig. 1B is identified as the methyl ester of vernolic acid. This finding thus demonstrates that the *E. lagascae* cytochrome P450 corresponding to EST eellc.pk002.i4 functions as a Δ¹²-linoleic acid epoxygenase in the biosynthesis of vernolic acid.

Supplementation of the growth media with α-linolenic acid resulted in the production of a second novel fatty acid by cells expressing the *E. lagascae* cytochrome P450 corresponding to EST eellc.pk002.i4. This methyl ester of this fatty acid, which is labeled as "Epoxy2" in Figure 1D, displayed a longer retention time than that of methyl vemolic acid [Figure 1E]. In addition, the mass spectra of trimethylsilyl derivatives of acidic methanol products of "Epoxy2" were consistent with those arising from a C₁₈ fatty acid methyl ester containing a Δ¹² epoxy group and two double bonds [Figure 2E-F]. Given that the precursor of this product is 18:3Δ^{9,12,15} (α-linolenic acid). "Epoxy2" is thus tentatively identified as the methyl ester of Δ¹²-epoxyoctadeca-9,15-dienoic acid (Δ¹²-epoxy-18:2Δ^{9,15}). This result demonstrates that the *E. lagascae* cytochrome P450 corresponding to EST eellc.pk002.i4 is also able to catalyze the Δ¹² epoxidation of α-linolenic acid.

### EXAMPLE 8

### Expression of the cDNA for Euphorbia lagascae EST eellc.pk002.i4 in Transgenic Tobacco Cells

The *Euphorbia lagascae* cytochrome P450 enzyme corresponding to the cDNA for EST eellc.pk002.i4 was expressed in tobacco (*Nicotiana tabacum*) callus under the control of the cauliflower mosaic virus 35S promoter in order to assess its function in transgenic plant cells. For these studies, the open-reading frame of the cDNA for EST eellc.pk002.i4 was initially amplified by PCR to generate flanking *Eco*RI and *Bam*HI sites for cloning into the plant expression vector. The sequence of the sense oligonucleotide used in the amplification reaction was 5'-gcggccgcgaattcGGAAAATGGAGCAGAAAAATC-3', SEQ ID NO:6; and the sequence of the antisense oligonucleotide was 5'-gcggccgcggatccTTAGAACATCGTTAATTAAAG-3', SEQ ID NO:7 (Note: the bases in lower case contain the added restriction sites and flanking sequence to facilitate restriction enzyme digestion). The design of the PCR primers was based on the sequence of the cDNA for EST eellc.pk002.i4 shown in SEQ ID NO:1. Thirty cycles of PCR amplification were conducted in a 100 µl volume using *Pfu* polymerase (Stratagene), and the template consisted of the cDNA for EST eellc.pk002.i4 in pBluescript SK (-). The product from this reaction was subcloned into pCR-Script AMP (Stratagene). Following restriction digestion with *Eco*RI and *Bam*HI*,* the PCR product was moved from pCR-Script AMP to the corresponding sites of vector pART7 [Gleave, A.P. (1992) *Plant Mol. Biol.* 20: 1203-1207]. The resulting construct consisted of the open reading frame of the cDNA for EST eellc.pk002.i4 fused at its 5' end to the cauliflower mosaic virus 35S promoter and at its 3' end to the transcriptional termination portion of the octopine synthase gene. These three sequence elements (promoter/EST eellc.pk002.i4 open-reading frame/3' *ocs* termination sequence) were then moved as a *Not*I fragment into the corresponding site of the binary vector pART27, which contains a kanamycin resistance marker for selection of transformed plant cells [Gleave, A.P. (1992) *Plant Mol. Biol.* 20: 1203-1207]. The resulting plasmid was designated pElCytP450. A vector control was prepared by the insertion of promoter and termination elements from pART7 into the *NotI* site of pART27. The resulting plasmid was designated pART27/35S. Plasmids pElCytP450 and pART27/35S were then transformed into *Agrobacterium tumefaciens* LBA4404 cells. Cultures derived from these cells were used for transformation of tobacco (*Nicotiana tabacum* cv. Xanthi) leave disks according to the protocol described by Rogers, S.G., Horsch, R.B., and Fraley, R.T. (1986) *Methods Enzymol.* 118: 627-648. Transgenic callus derived from these transformations was selected based on kanamycin resistance conferred by the binary vector. In addition, the presence of the transgene in this tissue was confirmed by Northern blot analysis.

The fatty acid composition of kanamycin-resistant tobacco callus obtained from leaf disk transformations was examined by gas chromatography (GC). The tissue used in these analyses was callus that had been transferred to fresh selection media and grown for an additional 3 to 4 weeks. To obtain fatty acid methyl esters for GC analyses, approximately 150 mg of transgenic tobacco callus was homogenized in 1 ml of a 1% (w/v) sodium methoxide in methanol using methods described by Hitz *et al.* (1994) *Plant Physiol.* 105:635-641. The resulting fatty acid methyl esters were analyzed using a Hewlett-Packard 5890 gas chromatograph fitted with an Omegawax 320 column (30 m x 0.32 mm inner diameter; Supelco). The oven temperature was programmed from 180°C (4 min hold) to 215°C at a rate of 5°C/min and then to 240°C at a rate of 20°C/min (0.5 min hold). To provide additional structural characterization, the mass spectra of acid-derivatized fatty acid methyl esters from the transgenic tobacco callus was determined using gas chromatography-mass spectrometry (GC-MS) as described in Example 7. Using these analytical methods, two fatty acid methyl esters were detected in tobacco callus transformed with pElCytP450 that were absent from vector control (pART/35S) callus [Figure 3]. Peaks corresponding to these fatty acids are labeled "Epoxy1" and "Epoxy 2" in the gas chromatogram shown in [Figure 3B]. The methyl ester of Epoxy 1 displayed a gas chromatographic retention time identical to that of methyl vemolic acid (Δ¹²-epoxy-18:1Δ^{9cis}) in extracts of *Euphorbia lagascae* seeds (Fig. 3C). In addition, acid derivitization and silylation of the methyl ester of Epoxy1 yielded two compounds with mass spectra identical to those obtained from a similar derivitization of methyl vemolic acid (see Example 7). Epoxy1 is thus identified as vemolic acid. The fatty acid methyl ester corresponding to Epoxy2 in Fig. 3B displayed the same retention time as the methyl ester of 12-epoxyoctadeca-9,15-dienoic acid (Δ¹²-epoxy-18:2Δ^{9,15}) described in Example 7. The mass spectra of compounds formed from the methanolic acid derivitization and silylation of the Epoxy2 methyl ester were identical to those of similarly prepared derivatives of methyl 12-epoxyoctadeca-9,15-dienoate as detailed in Example 7. Epoxy2 is thus identified as 12-epoxyoctadeca-9,15-dienoic acid. Based on substrate feeding studies with *Saccharomyces cerevisiae* described in Example 7, vernolic acid and Δ¹²-epoxy-18:2Δ^{9,15} in the transgenic tobacco cells result from the Δ¹² epoxygenase activity on linoleic (18:2Δ^{9,12}) and α-linolenic (18:3Δ^{9,12,15}) acids, respectively.

As summarized in Table 5, tobacco callus expressing the *Euphorbia lagascae* cytochrome P450 corresponding to EST eellc.pk002.i4 accumulated Δ¹² epoxy fatty acids (vemolic acid and Δ¹²-epoxy-18:2Δ^{9,15}) to amounts exceeding 15 wt% of the total fatty acids of the transgenic samples (TABLE 5).

These results thus demonstrate that the *Euphorbia lagascae* cytochrome P450 enzyme corresponding to EST eellc.pk002.i4 can be functionally expressed in transgenic plant cells to produce Δ¹² epoxy fatty acids.

**TABLE 5**

| Fatty Acid Compositions of Tobacco Callus Transformed with the pART27/35S Vector Lacking cDNA Insert (*pART27*/*35S*) or with the *Euphorbia lagascae* cDNA for EST eellc.pk002.i4 Behind a 35S Promoter (*pELCytP450*) | | |
|---|---|---|
| **Fatty Acid** | **pART27/35S (Vector Control)** (n=3) ¹ | **pElCytP450** (n=3) |
| | Weight% of Total Fatty Acids | |
| 16:0 | 18.8 ± 1.6 | 15.9 ± 0.9 |
| 18:0 | 4.0 ± 0.7 | 4.5 ± 0.7 |
| 18:1Δ⁹ | 2.7 ± 0.9 | 43.2 ± 2.7 |
| 18:2Δ^{9,12} | 46.4 ± 5.0 | 14.9 ± 1.7 |
| 18:3Δ^{9,12,15} | 27.1 ± 5.4 | 4.6 ± 0.5 |
| Δ¹²-Epoxy-18:1Δ⁹ | N.D.² | 13.3 ± 0.7 |
| (Vernolic Acid) | | |
| Δ¹²-Epoxy-18:2Δ^{9,15} | N.D. | 2.2 ± 0.2 |
| Other ³ | ≤1.0 | ≤1.5 |

| | | |
|---|---|---|
| ¹ Measurements from three independent samples of transgenic callus ± standard deviation. ² N.D., Not detected. ³ Includes 20:0, 20:1, 22:0, and 22:1. | | |

### EXAMPLE 9

### Production of Δ¹²-Epoxy Fatty Acids in Somatic Soybean Embryos

The *Euphorbia lagascae* cytochrome P450 enzyme corresponding to the cDNA for EST eellc.pk002.i4 was expressed in somatic soybean (*Glycine max*) embryos in order to examine its activity in a transgenic crop species. The open-reading frame of the cDNA for EST eellc.pk002.i4 was initially amplified by PCR using the same methodology and oligonucleotide primers [SEQ ID NO:6 and SEQ ID NO:7] as described in Example 8. The resulting PCR product was subcloned into the vector pCR-Script AMP (Stratagene) according to the manufacturer's protocol. The PCR product was then moved as a *Not*I fragment from pCR-Script AMP into the corresponding sites of the soybean expression vector pKS123 to generate the plasmid pKR31. Vector pKS123 is identical to the previously described vector pKS67 [World Patent Publication No. WO 00/11176] except that two *Asc*I restriction enzyme sites were added that flank the promoter and termination elements of the plant expression cassette. Plasmid pKR31 contains the open-reading frame of the cDNA for EST eellc.pk002.i4 fused on its 5' terminus with the promoter of the gene for the α'-subunit of β-conglycinin [Beachy, R.N. et al. (1985) *EMBO J.* 4:3047-3053] and on its 3' end with the termination elements from the phaseolin gene [Doyle, J.J. et al. (1986) *J. Biol. Chem.* 261:9228-9238]. The promoter of the gene for the α'-subunit of the β-conglycinin used in pKR31 directs the strong seed-specific expression of transgenes. Bacterial selection in vector pKR31 is conferred by a hygromycin B phosphotransferase gene [Gritz, L. and Davies, J. (1983) *Gene* 25:179-188] under control of the T7 RNA polymerase promoter, and plant selection is conferred by a second hygromycin B phosphotransferase gene under control of the cauliflower mosaic virus 35S promoter. Plasmid pKR31 was used for transformation of somatic soybean embryos as described below.

To induce somatic embryos, cotyledons, 3-5 mm in length dissected from surface sterilized, immature seeds of a soybean cultivar A2872 were cultured in the light or dark at 26°C on an appropriate agar medium for 6-10 weeks. Somatic embryos that produce secondary embryos were then excised and placed into a suitable liquid medium. After repeated selection for clusters of somatic embryos that multiplied as early, globular staged embryos, the suspensions were maintained as described below.

Soybean embryogenic suspension cultures were maintained in 35 mL liquid media on a rotary shaker, 150 rpm, at 26°C with florescent lights on a 16:8 hour day/night schedule. Cultures were subcultured every two weeks by inoculating approximately 35 mg of tissue into 35 mL of liquid medium.

Soybean embryogenic suspension cultures were then transformed with pKR31 by the method of particle gun bombardment (Klein, T.M. et al. (1987) *Nature* (London) 327:70, U.S. Patent No. 4,945,050). A Du Pont Biolisticä PDS1000/HE instrument (helium retrofit) was used for these transformations.

To 50 mL of a 60 mg/mL 1 mm gold particle suspension were added (in order): 5 mL DNA (1 mg/mL), 20 ml spermidine (0.1 M), and 50 mL CaCl₂ (2.5 M). The particle preparation was then agitated for three minutes, spun in a microfuge for 10 seconds and the supernatant removed. The DNA-coated particles were then washed once in 400 mL 70% ethanol and resuspended in 40 mL of anhydrous ethanol. The DNA/particle suspension was sonicated three times for one second each. Five mL of the DNA-coated gold particles was then loaded on each macro carrier disk.

Approximately 300-400 mg of a two-week-old suspension culture was placed in an empty 60x15-mm petri dish and the residual liquid removed from the tissue with a pipette. For each transformation experiment, approximately 5 to 10 plates of tissue were bombarded. Membrane rupture pressure was set at 1100 psi and the chamber was evacuated to a vacuum of 28 inches mercury. The tissue was placed approximately 3.5 inches away from the retaining screen and bombarded three times. Following bombardment, the tissue was divided in half and placed back into liquid and cultured as described above.

Five to seven days post bombardment, the liquid media was exchanged with fresh media, and eleven to twelve days post bombardment with fresh media containing 50 mg/mL hygromycin. This selective media was refreshed weekly. Seven to eight weeks post bombardment, green, transformed tissue was observed growing from untransformed, necrotic embryogenic clusters. Isolated green tissue was removed and inoculated into individual flasks to generate new, clonally propagated, transformed embryogenic suspension cultures. Each new line was treated as an independent transformation event. These suspensions were then subcultured and maintained as clusters of immature embryos. Immature embryos at this stage produce storage products, including storage lipids that are similar in composition to zygotic embryos at a similar stage of development (see World Patent Publication No. WO 94/11516). Expression of the *Euphorbia lagascae* cytochrome P450 transgene in the hygromycin-selected embryo lines was confirmed by PCR amplification using sequence-specific primers and first-strand cDNA prepared from total RNA isolated from the transgenic embryos.

Transgenic somatic soybean embryos selected and maintained in this manner were analyzed for Δ¹²-epoxy fatty acid accumulation using gas chromatography (GC) or gas chromatography-mass spectrometry (GC-MS). Individual embryos expressing the *Euphorbia lagascae* cytochrome P450 epoxygenase corresponding to EST eellc.pk002.i4 were homogenized in 1% (w/v) sodium methoxide in methanol (as described in Example 7) to generate fatty acid methyl esters from the total lipids of these tissues. Fatty acid methyl esters resulting from this transesterification step were analyzed by GC and GC-MS using methods described in Example 7. In somatic embryos expressing the cDNA for EST eellc.pk002.i4, two novel fatty acid methyl esters were detected that were absent from untransformed embryos [Figure 4]. Gas chromatographic peaks corresponding to these fatty acid methyl esters are designated "Epoxy1" and "Epoxy2" in [Figure 4B]. The fatty acid methyl esters corresponding to Epoxy1 and Epoxy2 had retention times identical to those of methyl vemolic acid and Δ¹²-epoxy-18:2Δ^{9,15}, respectively. In addition, the mass spectra obtained following methanolic sulfuric acid derivitization (as described in Example 7) of these fatty acid methyl esters were identical with those of derivatives of methyl vemolic acid and Δ¹²-epoxy-18:2Δ^{9,15} prepared in a similar manner. Thus, the two novel fatty acids produced by somatic soybean embryos expressing the cDNA for EST eellc.pk002.i4 are identified as vemolic acid (Δ¹²-epoxy-18:1Δ⁹) and Δ¹²-epoxy-18:2Δ^{9,15}. Based on substrate feeding studies described in Example 7, these fatty acids arise from the modification of the Δ¹² double bond of linoleic and α-linolenic acids. These results further demonstrate that the cytochrome P450 enzyme encoded by the cDNA for EST eellc.pk002.i4 functions *in planta* as a Δ¹² fatty acid epoxygenase. These results also demonstrate the ability to produce epoxy fatty acids in a crop species, such as soybean, via the expression of the cDNA for EST eellc.pk002.i4.

Table 6 shows a comparison of the fatty acid compositions of untransformed somatic soybean embryos and embryos from transgenic line MSE578-6-9 transformed with the *Euphorbia lagascae* cytochrome P450 cDNA corresponding to EST eellc.pk002.i4. As indicated, epoxy fatty acids accumulate to amounts >7 wt% of the total fatty acids of the transgenic embryo lines but are not detected in untransformed lines.

**TABLE6**

| Fatty Acid Compositions From Untransformed Somatic Soybean Embryos and Transgenic Somatic Soybean Embryo Line MSE578-6-9 Expressing the *Euphorbia lagascae* Cytochrome P450 cDNA Corresponding to EST eellc.pk002.i4 | | |
|---|---|---|
| Fatty Acid | Untransformed (n=3) ¹ | Line MSE578-6-9 (+*E. lagascae* Cyt P450) (n=3) |
| | Weight% of Total Fatty Acids | |
| 16:0 | 14.2 ± 0.2 | 13.4 ± 0.6 |
| 18:0 | 3.4 ± 0.5 | 3.9 ± 1.0 |
| 18:1Δ⁹ | 8.7 ± 0.7 | 10.5 ± 1.9 |
| 18:2Δ^{9,12} | 53.7 ± 3.9 | 46.9 ± 1.9 |
| 18:3Δ^{9,12,15} | 19.1 ± 3.2 | 17.1 ± 2.1 |
| Vernolic | N.D.² | 5.4 ± 0.4 |
| (Δ¹²-Epoxy- | | |
| 18:1Δ⁹) | | |
| Δ¹²-Epoxy- | N.D. | 1.8 ± 0.1 |
| 18:2Δ^{9,15} | | |
| Other³ | ≤0.9 | ≤1.0 |

| | | |
|---|---|---|
| ¹ Values from three separate measurements (± standard deviation) of single embryos ² N.D., Not detected. ³ Includes 20:0, 20:1, and 22:0. | | |

## Claims

1. An isolated polynucleotide comprising a nucleotide sequence selected from the group consisting of:
(a) a first nucleotide sequence encoding a polypeptide which is a cytochrome P450 enzyme associated with the synthesis of delta 12-epoxy fatty acids wherein said polypeptide has at least 50% identity based on the Clustal method of alignment when compared to a polypeptide of SEQ ID NO:2; and
(b) a second nucleotide sequence comprising a complement of the first nucleotide sequence.

2. An isolated polynucleotide as claimed in claim 1, wherein in part (a), the polypeptide has at least 55% identity based on the Clustal method of alignment when compared to a polypeptide of SEQ ID NO: 2.

3. An isolated polynucleotide as claimed in claim 1, wherein in part (a), the polypeptide has at least 60% identity based on the Clustal method of alignment when compared to a polypeptide of SEQ ID NO: 2.

4. An isolated polynucleotide as claimed in claim 1, wherein in part (a), the polypeptide has at least 65% identity based on the Clustal method of alignment when compared to a polypeptide of SEQ ID NO: 2.

5. An isolated polynucleotide as claimed in claim 1, wherein in part (a), the polypeptide has at least 70% identity based on the Clustal method of alignment when compared to a polypeptide of SEQ ID NO: 2.

6. An isolated polynucleotide as claimed in claim 1, wherein in part (a), the polypeptide has at least 75% identity based on the Clustal method of alignment when compared to a polypeptide of SEQ ID NO: 2.

7. An isolated polynucleotide as claimed in claim 1, wherein in part (a), the polypeptide has at least 80% identity based on the Clustal method of alignment when compared to a polypeptide of SEQ ID NO: 2.

8. An isolated polynucleotide as claimed in claim 1, wherein in part (a), the polypeptide has at least 85% identity based on the Clustal method of alignment when compared to a polypeptide of SEQ ID NO: 2.

9. An isolated polynucleotide as claimed in claim 1, wherein in part (a), the polypeptide has at least 90% identity based on the Clustal method of alignment when compared to a polypeptide of SEQ ID NO: 2.

10. An isolated polynucleotide as claimed in claim. 1, wherein in part (a), the polypeptide has at least 95% identity based on the Clustal method of alignment when compared to a polypeptide of SEQ ID NO: 2.

11. A chimeric construct comprising the isolated polynucleotide of any one of claims 1 to 10 operably linked to at least one suitable regulatory sequence.

12. An isolated host cell comprising the chimeric construct of Claim 11.

13. An isolated host cell comprising an isolated polynucleotide of any one of claims 1 to 10.

14. The host cell of Claim 13 wherein the host cell is selected from the group consisting of yeast, bacteria, and plant

15. A method of selecting an isolated polynucleotide that affects the level of delta-12 epoxy fatty acids in a host cell, the method comprising the steps of:
(a) making an isolated polynucleotide comprising the polynucleotide sequence of any one of claims 1 to 10;
(b) introducing the isolated polynucleotide into a host cell;
(c) determining presence or absence of delta-12 epoxy fatty acids in the host cell of (b).

16. The method of Claim 15 wherein the isolated polynucleotide consists of a nucleotide sequence of SEQ ID NO:1.

17. A method of obtaining a nucleic acid fragment encoding a cytochrome P450 enzyme associated with the synthesis of delta-12 epoxy fatty acids comprising the steps of:
(a) probing a cDNA or genomic library with an isolated polynucleotide comprising the nucleotide sequence of SEQ ID NO:1 and a complement of such nucleotide sequences;
(b) identifying a DNA clone that hybridizes with the polynucleotide of (a) or the complement thereof;
(c) isolating the identified DNA clone; and
(d) sequencing a cDNA or genomic fragment that comprises the isolated DNA clone.

18. A method for producing delta-12 epoxy fatty acids in a host cell which comprises:
(a) transforming a host cell with the chimeric construct of Claim 11;
(b) growing the transformed host cells of step (a); and
(c) determining the presence or absence of delta-12 epoxy fatty acids in the transformed cells of (b).

## Patentansprüche

1. Isoliertes Polynukleotid, umfassend eine Nukleotidsequenz, die aus der Gruppe ausgewählt ist, bestehend aus:
(a) einer ersten Nukleotidsequenz, kodierend ein Polypeptid, bei dem es sich um ein Cytochrom-P450-Enzym handelt, das mit der Synthese von Δ-12-Epoxyfettsäuren assoziiert ist, worin das Polypeptid eine mindestens 50%ige Identität bezogen auf das Clustal-Alignmentverfahren aufweist, wenn es mit einem Polypeptid von SEQ ID NO:2 verglichen wird; und
(b) einer zweiten Nukleotid-Sequenz, umfassend ein Komplement von der ersten Nukleotidsequenz.

2. Isoliertes Polynukleotid nach Anspruch 1, worin in Teil (a) das Polypeptid eine mindestens 55%ige Identität bezogen auf das Clustal-Alignmentverfahren aufweist, wenn es mit einem Polypeptid von SEQ ID NO:2 verglichen wird.

3. Isoliertes Polynukleotid nach Anspruch 1, worin in Teil (a) das Polypeptid eine mindestens 60%ige Identität bezogen auf das Clustal-Alignmentverfahren aufweist, wenn es mit einem Polypeptid von SEQ ID NO:2 verglichen wird.

4. Isoliertes Polynukleotid nach Anspruch 1, worin in Teil (a) das Polypeptid eine mindestens 65%ige Identität bezogen auf das Clustal-Alignmentverfahren aufweist, wenn es mit einem Polypeptid von SEQ ID NO:2 verglichen wird.

5. Isoliertes Polynukleotid nach Anspruch 1, worin in Teil (a) das Polypeptid eine mindestens 70%ige Identität bezogen auf das Clustal-Alignmentverfahren aufweist, wenn es mit einem Polypeptid von SEQ ID NO:2 verglichen wird.

6. Isoliertes Polynukleotid nach Anspruch 1, worin in Teil (a) das Polypeptid eine mindestens 75%ige Identität bezogen auf das Clustal-Alignmentverfahren aufweist, wenn es mit einem Polypeptid von SEQ ID NO:2 verglichen wird.

7. Isoliertes Polynukleotid nach Anspruch 1, worin in Teil (a) das Polypeptid eine mindestens 80%ige Identität bezogen auf das Clustal-Alignmentverfahren aufweist, wenn es mit einem Polypeptid von SEQ ID NO:2 verglichen wird.

8. Isoliertes Polynukleotid nach Anspruch 1, worin in Teil (a) das Polypeptid eine mindestens 85%ige Identität bezogen auf das Clustal-Alignmentverfahren aufweist, wenn es mit einem Polypeptid von SEQ ID NO:2 verglichen wird.

9. Isoliertes Polynukleotid nach Anspruch 1, worin in Teil (a) das Polypeptid eine mindestens 90%ige Identität bezogen auf das Clustal-Alignmentverfahren aufweist, wenn es mit einem Polypeptid von SEQ ID NO:2 verglichen wird.

10. Isoliertes Polynukleotid nach Anspruch 1, worin in Teil (a) das Polypeptid eine mindestens 95%ige Identität bezogen auf das Clustal-Alignmentverfahren aufweist, wenn es mit einem Polypeptid von SEQ ID NO:2 verglichen wird.

11. Chimäres Konstrukt, umfassend das isolierte Polynukleotid nach einem der Ansprüche 1 bis 10, das mit mindestens einer geeigneten Regulationssequenz operativ verknüpft ist.

12. Isolierte Wirtszelle, umfassend das chimäre Konstrukt nach Anspruch 11.

13. Isolierte Wirtszelle, umfassend ein isoliertes Polynukleotid nach einem der Ansprüche 1 bis 10.

14. Wirtszelle nach Anspruch 13, worin die Wirtszelle aus der Gruppe ausgewählt ist, bestehend aus Hefe, Bakterien und Pflanzen.

15. Verfahren des Auswählens eines isolierten Polynukleotids, das sich auf die Konzentration der Δ-12-Epoxyfettsäuren in einer Wirtszelle auswirkt, wobei das Verfahren die folgenden Schritte umfasst:
(a) Herstellen eines isolierten Polynukleotids, umfassend die Polynukleotidsequenz nach einem der Ansprüche 1 bis 10;
(b) Einführen des isolierten Polynukleotids in eine Wirtszelle;
(c) Bestimmen der An- oder Abwesenheit von Δ-12-Epoxyfettsäuren in der Wirtszelle von (b).

16. Verfahren nach Anspruch 15, worin das isolierte Polynukleotid aus einer Nukelotidsequenz von SEQ ID NO: 1 besteht.

17. Verfahren zum Erhalt eines Nukleinsäurefragments, kodierend ein Cytochrom-P450-Enzym, das mit der Synthese von Δ-12-Epoxyfettsäuren assoziiert ist, umfassend die folgenden Schritte:
(a) Sondieren einer cDNA oder genomischen Bibliothek mit einem isolierten Polynukleotid, umfassend die Nukleotidsequenz von SEQ ID NO:1 und ein Komplement von solchen Nukleotidsequenzen;
(b) Identifizieren eines DNA-Klons, der mit dem Polynukleotid von (a) oder dem Komplement davon hybridisiert;
(c) Isolieren des identifizierten DNA-Klons; und
(d) Sequenzieren einer cDNA oder eines genomischen Fragments, das den isolierten DNA-Klon umfasst.

18. Verfahren zur Herstellung von Δ-12-Epoxyfettsäuren in einer Wirtszelle, die Folgendes umfasst:
(a) Transformieren einer Wirtszelle mit dem chimären Konstrukt nach Anspruch 11;
(b) Züchten der transformierten Wirtszellen von Schritt (a); und
(c) Bestimmen der An- oder Abwesenheit von Δ-12-Epoxyfettsäuren in den transformierten Zellen von (b).

## Revendications

1. Polynucléotide isolé comprenant une séquence de nucléotides sélectionnée dans le groupe constitué de:
(a) une première séquence de nucléotides codant pour un polypeptide qui est une enzyme cytochrome P450 associée à la synthèse d'acides gras delta-12 époxy où ledit polypeptide a au moins 50 % d'identité basée sur la méthode d'alignement Clustal lorsqu'il est comparé à un polypeptide de SEQ ID NO:2; et
(b) une seconde séquence de nucléotides comprenant un complément de la première séquence de nucléotides.

2. Polynucléotide isolé selon la revendication 1, dans lequel dans la partie (a), le polypeptide a au moins 55 % d'identité basée sur la méthode d'alignement Clustal lorsqu'il est comparé à un polypeptide de SEQ ID NO:2.

3. Polynucléotide isolé selon la revendication 1, dans lequel dans la partie (a), le polypeptide a au moins 60 % d'identité basée sur la méthode d'alignement Clustal lorsqu'il est comparé à un polypeptide de SEQ ID NO:2.

4. Polynucléotide isolé selon la revendication 1, dans lequel dans la partie (a), le polypeptide a au moins 65 % d'identité basée sur la méthode d'alignement Clustal lorsqu'il est comparé à un polypeptide de SEQ ID NO:2.

5. Polynucléotide isolé selon la revendication 1, dans lequel dans la partie (a), le polypeptide a au moins 70 % d'identité basée sur la méthode d'alignement Clustal lorsqu'il est comparé à un polypeptide de SEQ ID NO:2.

6. Polynucléotide isolé selon la revendication 1, dans lequel dans la partie (a), le polypeptide a au moins 75 % d'identité basée sur la méthode d'alignement Clustal lorsqu'il est comparé à un polypeptide de SEQ ID NO:2.

7. Polynucléotide isolé selon la revendication 1, dans lequel dans la partie (a), le polypeptide a au moins 80 % d'identité basée sur la méthode d'alignement Clustal lorsqu'il est comparé à un polypeptide de SEQ ID NO:2.

8. Polynucléotide isolé selon la revendication 1, dans lequel dans la partie (a), le polypeptide a au moins 85 % d'identité basée sur la méthode d'alignement Clustal lorsqu'il est comparé à un polypeptide de SEQ ID NO:2.

9. Polynucléotide isolé selon la revendication 1, dans lequel dans la partie (a), le polypeptide a au moins 90 % d'identité basée sur la méthode d'alignement Clustal lorsqu'il est comparé à un polypeptide de SEQ ID NO:2.

10. Polynucléotide isolé selon la revendication 1, dans lequel dans la partie (a), le polypeptide a au moins 95 % d'identité basée sur la méthode d'alignement Clustal lorsqu'il est comparé à un polypeptide de SEQ ID NO:2.

11. Construction chimère comprenant le polynucléotide isolé selon l'une quelconque des revendications 1 à 10 lié de manière fonctionnelle à au moins une séquence régulatrice adaptée.

12. Cellule hôte isolée comprenant la construction chimère selon la revendication 11.

13. Cellule hôte isolée comprenant un polynucléotide isolé selon l'une quelconque des revendications 1 à 10.

14. Cellule hôte selon la revendication 13 dans laquelle la cellule hôte est sélectionnée dans le groupe constitué de levures, de bactéries, et de plantes.

15. Procédé de sélection d'un polynucléotide isolé qui affecte le niveau d'acides gras delta-12 époxy dans une cellule hôte, le procédé comprenant les étapes de:
(a) fabrication d'un polynucléotide isolé comprenant la séquence de polynucléotide selon l'une quelconque des revendications 1 à 10;
(b) l'introduction du polynucléotide isolé dans une cellule hôte;
(c la détermination de la présence ou de l'absence d'acides gras delta-12 époxy dans la cellule hôte de (b).

16. Procédé selon la revendication 15, dans lequel le polynucléotide isolé consiste en une séquence de nucléotides de SEQ ID NO: 1.

17. Procédé d'obtention d'un fragment d'acide nucléique codant pour une enzyme cytochrome P450 associée à la synthèse d'acides gras delta-12 époxy comprenant les étapes de:
(a) l'examen d'une banque génomique ou d'ADNc avec un polynucléotide isolé comprenant la séquence de nucléotides de SEQ ID NO: 1 et un complément de telles séquences de nucléotides;
(b) l'identification d'un clone d'ADN qui s'hybride avec le polynucléotide de
(a) ou le complément de celui-ci;
(c) l'isolement du clone d'ADN identifié; et
(d) le séquençage d'un fragment génomique ou d'ADNc qui comprend le clone d'ADN isolé.

18. Procédé de production d'acides gras delta-12 époxy dans une cellule hôte qui comprend:
(a) la transformation d'une cellule hôte avec la construction chimère selon la revendication 11;
(b) la croissance des cellules hôtes transformées de l'étape (a); et
(c) la détermination de la présence ou de l'absence d'acides gras delta-12 époxy dans les cellules transformées de (b).


### SEQUENCE LISTING

<110> E.I. du Pont de Nemours and Company
<120> A Cytochrome P450 enzyme associated with the synthesis of Δ¹²-epoxy fatty acids
<130> BB1465 PCT
<140>
   <141>
<150> 60/219833
   <151> July 21, 2000
<160> 7
<170> Microsoft Office 97
<210> 1
   <211> 1733
   <212> DNA
   <213> Euphorbia lagascae
<400> 1
   gcataaaagg aaaatggagc agaaaaatct ctcttttccg agcattttaa taagttttct   60
   gcttgtttta atcttagtag tagtcatgag gttgtggaag aaacagaatc cacctccagg  120
   gccatggaag tttcctatca taggtaatct tcctcattta ttactcactt ctgatctagg  180
   ccatgaacgt tttagagcct tggctcaaat ttatggacct gttatgagtc ttcaaattgg  240
   ccaagtttca gctgttgtca tttcttcagc tgaagcagcc aaagaggtta tgaaaactca  300
   ggctgatgcc ttcgcccaac gccctatcgt cttggacgca cagattgtgt tttataatcg  360
   gaaagatgtc ttgtttgctt catatggaga tcactggagg cagatgaaga aaatttggat  420
   acttgaattt ctgagtgcca aaaaagttca atcctccagg ttaatccgag aggaagaaat  480
   ggaggatgcc atcacattcc tccgttcgaa agccggatct ccggtcaata ttacaaagat  540
   catttatggc attataattt cgatcatgat aagaacatcc gttggtaatt gtaagcaaaa  600
   agaaagattg ctgagtgttg ccgatgcagt caatgaggca gcgacgagtt ttggcaccgc  660
   agacgctttt ccgacgtgga aattacttca ctatatcatt ggagctgagt caaaacccag  720
   gcgtttgcat caggagattg acgatatact tgaagagatt cttaatgaac acaaagccaa  780
   taagcctttt gaagcggata acttaatgga tgttctattg aatcttcaaa aaaatggaaa  840
   cgttccagtg ccagtgacaa acgaaagcat caaagcatcc gttttgcaaa tgtttactgc  900
   cgggagcgaa acaacttcga aagctacaga atgggtaatg gcagagctga tgaaaaatcc  960
   aactgaacta agaaaagcac aagaagaagt tagacaagta tttggtgaaa tgggaaaagt 1020
   tgatgaatca agatttcatg atttgaaatt cttcaagtta gtggttaaag aaactctaag 1080
   attacatcct ccggttgtct tgattccgag ggagtgtaga gaaacaacac gaattgatgg 1140
   atatgaaatt catccgaaca ctcgaattgt tgtgaatgct tgggcgatag gaagagatcc 1200
   taatacttgg tcggaacctg gaaagtttaa cccagaaagg tttaaagatt gtgcaattga 1260
   ttataaaggg acgacatttg aactggtacc atttggtgca ggaaaaagaa tatgtcctgg 1320
   cattacttca gctattacca atttggagta tgtcattata aatctattat atcattttaa 1380
   ttgggaactg gccgatggaa ttacacctca aacacttgat atgactgaag ctattggcgg 1440
   tgctctcagg aaaaaaatag atcttaagtt gattcctatt ccatatcaag ttagcttagg 1500
   ctcaaatatt tcttgattac ataggagggt tgaaatatat ataataaact ttaattaacg 1560
   atgttctaat atggtttggg tgagttataa taggttttcc accgatcata taagtagcct 1620
   tctttgatgg atgggttaga ttataatgag ttgtgggttg gatttttaga tgggttaaat 1680
   gatttggatg gataataata aattgaaatg ttttcttttt caaatccgaa aaa        1733
<210> 2
   <211> 500
   <212> PRT
   <213> Euphorbia lagascae
<400> 2
   Met Glu Gln Lys Asn Leu Ser Phe Pro Ser Ile Leu Ile Ser Phe Leu
   1 5 10 15
   Leu Val Leu Ile Leu Val Val Val Met Arg Leu Trp Lys Lys Gln Asn
   20 25 30
   Pro Pro Pro Gly Pro Trp Lys Phe Pro Ile Ile Gly Asn Leu Pro His
   35 40 45
   Leu Leu Leu Thr Ser Asp Leu Gly His Glu Arg Phe Arg Ala Leu Ala
   50 55 60
   Gln Ile Tyr Gly Pro Val Met Ser Leu Gln Ile Gly Gln Val Ser Ala
   65 70 75 80
   Val Val Ile Ser Ser Ala Glu Ala Ala Lys Glu Val Met Lys Thr Gln
   85 90 95
   Ala Asp Ala Phe Ala Gln Arg Pro Ile Val Leu Asp Ala Gln Ile Val
   100 105 110
   Phe Tyr Asn Arg Lys Asp Val Leu Phe Ala Ser Tyr Gly Asp His Trp
   115 120 125
   Arg Gln Met Lys Lys Ile Trp Ile Leu Glu Phe Leu Ser Ala Lys Lys
   130 135 140
   Val Gln Ser Ser Arg Leu Ile Arg Glu Glu Glu Met Glu Asp Ala Ile
   145 150 155 160
   Thr Phe Leu Arg Ser Lys Ala Gly Ser Pro Val Asn Ile Thr Lys Ile
   165 170 175
   Ile Tyr Gly Ile Ile Ile Ser Ile Met Ile Arg Thr Ser Val Gly Asn
   180 185 190
   Cys Lys Gln Lys Glu Arg Leu Leu Ser Val Ala Asp Ala Val Asn Glu
   195 200 205
   Ala Ala Thr Ser Phe Gly Thr Ala Asp Ala Phe Pro Thr Trp Lys Leu
   210 215 220
   Leu His Tyr Ile Ile Gly Ala Glu Ser Lys Pro Arg Arg Leu His Gln
   225 230 235 240
   Glu Ile Asp Asp Ile Leu Glu Glu Ile Leu Asn Glu His Lys Ala Asn
   245 250 255
   Lys Pro Phe Glu Ala Asp Asn Leu Met Asp Val Leu Leu Asn Leu Gln
   260 265 270
   Lys Asn Gly Asn Val Pro Val Pro Val Thr Asn Glu Ser Ile Lys Ala
   275 280 285
   Ser Val Leu Gln Met Phe Thr Ala Gly Ser Glu Thr Thr Ser Lys Ala
   290 295 300
   Thr Glu Trp Val Met Ala Glu Leu Met Lys Asn Pro Thr Glu Leu Arg
   305 310 315 320
   Lys Ala Gln Glu Glu Val Arg Gln Val Phe Gly Glu Met Gly Lys Val
   325 330 335
   Asp Glu Ser Arg Phe His Asp Leu Lys Phe Phe Lys Leu Val Val Lys
   340 345 350
   Glu Thr Leu Arg Leu His Pro Pro Val Val Leu Ile Pro Arg Glu Cys
   355 360 365
   Arg Glu Thr Thr Arg Ile Asp Gly Tyr Glu Ile His Pro Asn Thr Arg
   370 375 380
   Ile Val Val Asn Ala Trp Ala Ile Gly Arg Asp Pro Asn Thr Trp Ser
   385 390 395 400
   Glu Pro Gly Lys Phe Asn Pro Glu Arg Phe Lys Asp Cys Ala Ile Asp
   405 410 415
   Tyr Lys Gly Thr Thr Phe Glu Leu Val Pro Phe Gly Ala Gly Lys Arg
   420 425 430
   Ile Cys Pro Gly Ile Thr Ser Ala Ile Thr Asn Leu Glu Tyr Val Ile
   435 440 445
   Ile Asn Leu Leu Tyr His Phe Asn Trp Glu Leu Ala Asp Gly Ile Thr
   450 455 460
   Pro Gln Thr Leu Asp Met Thr Glu Ala Ile Gly Gly Ala Leu Arg Lys
   465 470 475 480
   Lys Ile Asp Leu Lys Leu Ile Pro Ile Pro Tyr Gln Val Ser Leu Gly
   485 490 495
   Ser Asn Ile Ser
   500
<210> 3
   <211> 502
   <212> PRT
   <213> Capsicum annuum
<400> 3
   Met Glu Ile Gln Phe Thr Asn Leu Val Ala Phe Leu Leu Phe Leu Ser
    1               5                  10                  15
   Ser Ile Ile Leu Leu Leu Lys Lys Trp Lys Thr Gln Lys Leu Asn Leu
               20                  25                  30
   Pro Pro Gly Pro Trp Lys Leu Pro Phe Ile Gly Ser Leu His His Leu
           35                  40                  45
   Ala Val Ala Gly Pro Leu Pro His His Gly Leu Lys Asn Leu Ala Lys
       50                  55                  60
   Leu Tyr Gly Pro Leu Met His Leu Arg Leu Gly Glu Ile Pro Thr Val
   65                  70                  75                  80
   Ile Ile Ser Ser Pro Arg Met Ala Lys Glu Val Leu Lys Thr His Asp
   85 90 95
   Leu Ala Phe Ala Thr Arg Pro Lys Leu Val Val Ala Asp Ile Val His
   100 105 110
   Tyr Asp Ser Thr Asp Ile Ala Phe Ser Pro Tyr Gly Glu Tyr Trp Arg
   115 120 125
   Gln Ile Arg Lys Ile Cys Ile Leu Glu Leu Leu Ser Ala Lys Met Val
   130 135 140
   Lys Phe Phe Ser Ser Ile Arg Gln Asp Glu Leu Ser Met Met Val Ser
   145 150 155 160
   Ser Ile Arg Thr Met Pro Asn Phe Pro Val Asn Leu Thr Asp Lys Ile
   165 170 175
   Phe Trp Phe Thr Ser Ser Val Thr Cys Arg Ser Ala Leu Gly Lys Ile
   180 185 190
   Cys Arg Asp Gln Asp Lys Leu Ile Ile Phe Met Arg Glu Ile Ile Ser
   195 200 205
   Leu Thr Gly Gly Phe Ser Ile Ala Asp Phe Phe Pro Thr Trp Lys Met
   210 215 220
   Leu His Asp Val Gly Gly Ser Lys Thr Arg Leu Leu Lys Ala His Arg
   225 230 235 240
   Lys Ile Asp Glu Ile Leu Glu His Val Val Asn Glu His Lys Gln Asn
   245 250 255
   Arg Ala Asp Gly Gln Lys Gly Asn Gly Glu Phe Gly Gly Glu Asp Leu
   260 265 270
   Ile Asp Val Leu Leu Arg Val Arg Glu Ser Gly Glu Val Gln Ile Ser
   275 280 285
   Ile Thr Asp Asp Asn Ile Lys Ser Ile Leu Val Asp Met Phe Ser Ala
   290 295 300
   Gly Ser Glu Thr Ser Ser Thr Thr Ile Ile Trp Ala Leu Ala Glu Met
   305 310 315 320
   Met Lys Lys Pro Ser Val Leu Ala Lys Ala Gln Ala Glu Val Arg Gln
   325 330 335
   Val Leu Lys Glu Lys Lys Gly Phe Gln Gln Ile Asp Leu Asp Glu Leu
   340 345 350
   Lys Tyr Leu Lys Leu Val Ile Lys Glu Thr Leu Arg Met His Pro Pro
   355 360 365
   Ile Pro Leu Leu Val Pro Arg Glu Cys Met Lys Asp Thr Lys Ile Asp
   370 375 380
   Gly Tyr Asn Ile Pro Phe Lys Thr Arg Val Ile Val Asn Ala Trp Ala
   385 390 395 400
   Ile Gly Arg Asp Pro Glu Ser Trp Asp Asp Pro Glu Ser Phe Ser Pro
                   405                 410                 415
   Glu Arg Phe Glu Asn Ser Ser Val Asp Phe Leu Gly Ser His His Gln
               420                 425                 430
   Phe Ile Pro Phe Gly Ala Gly Arg Arg Ile Cys Pro Gly Met Leu Phe
           435                 440                 445
   Gly Leu Ala Asn Val Gly Gln Pro Leu Ala Gln Leu Leu Tyr His Phe
       450                 455                 460
   Asp Arg Lys Leu Pro Asn Gly Gln Ser His Glu Asn Leu Asp Met Thr
   465                 470                 475                 480
   Glu Ser Pro Gly Ile Ser Ala Thr Arg Lys Asp Asp Leu Val Leu Ile
                   485                 490                 495
   Ala Thr Pro Tyr Asp Pro
               500
<210> 4
   <211> 51
   <212> DNA
   <213> synthetic construct
<400> 4
   tcaaggagaa aaaaccccgg atccatggag cagaaaaatc tctcttttcc g 51
<210> 5
   <211> 35
   <212> DNA
   <213> synthetic construct
<400> 5
   ggccagtgaa ttgtaatacg actcactata gggcg 35
<210> 6
   <211> 35
   <212> DNA
   <213> synthetic construct
<400> 6
   gcggccgcga attcggaaaa tggagcagaa aaatc 35
<210> 7
   <211> 35
   <212> DNA
   <213> synthetic construct
<400> 7
   gcggccgcgg atccttagaa catcgttaat taaag 35